# EUROPÄISCHE PATENTSCHRIFT

(11) **EP 2 012 742 B1**
(45) Veröffentlichungstag und Bekanntmachung des Hinweises auf die Patenterteilung: **19.03.2014**
(21) Anmeldenummer: 07724016.6
(22) Anmeldetag: 05.04.2007
(51) Int. Cl.: A61K 8/49, A61Q 5/06, A61Q 5/08, A61Q 5/10

(54) **AUFHELL- UND/ODER FÄRBEMITTEL MIT PIPERIDONEN**
BLEACHING AND/OR COLOURING AGENTS CONTAINING PIPERIDONES
PRODUITS ÉCLAIRCISSANTS ET/OU COLORANTS CONTENANT DES PIPÉRIDONES

(30) Priorität: 13.04.2006 DE 102006017835
(43) Veröffentlichungstag der Anmeldung: 14.01.2009
(73) Patentinhaber: Henkel AG & Co. KGaA, 40589 Düsseldorf (DE)
(72) Erfinder: HÖFFKES, Horst, 40595 Düsseldorf (US); FUHR, Denise, 40468 Düsseldorf (DE); GROSS, Wibke, 40549 Düsseldorf (DE); KNÜBEL, Georg, 40219 Düsseldorf (DE); AKRAM, Mustafa, 22457 Hamburg (DE); HOLLENBERG, Detlef, 40699 Erkrath (DE); REICHERT, Anja, 40629 Düsseldorf (DE)
(86) Internationale Anmeldenummer: PCT/EP2007/003075
(87) Internationale Veröffentlichungsnummer: WO 2007/118617

(56) Entgegenhaltungen:
- WO-A-03/029359
- WO-A-2005/110995
- GB-A- 2 018 302

## Beschreibung

Die vorliegende Erfindung betrifft Mittel zum Färben und/oder Aufhellen keratinischer Fasern, d.h. Mittel zur Anwendung auf Keratinfasern, insbesondere menschlichen Haaren, und deren Verwendung.

Gleichzeitig besteht aber auch vielfach der Wunsch, die Haarfarbe zu ändern und zusammen mit der Haarfärbung auch eine Aufhellung des zu färbenden Haares zu erreichen.

Konventionelle Haarfärbemittel bestehen in der Regel aus mindestens einer Entwickler- und mindestens einer Kupplersubstanz und enthalten ggf. noch direktziehende Farbstoffe als Nuanceure. Kuppler- und Entwicklerkomponenten werden auch als Oxidationsfarbstoffvorprodukte bezeichnet.

Vor ihrer Anwendung auf menschliches Haar werden Haarfärbe- und/oder -aufhellungsmittel in fester oder pastöser Form mit verdünnter wäßriger Wasserstoffperoxid-Lösung vermischt. Diese Mischung wird dann auf das Haar aufgebracht und nach einer bestimmten Einwirkzeit wieder ausgespült. Die Einwirkungsdauer auf dem Haar zur Erzielung einer vollständigen Ausfärbung bzw. Aufhellung liegt zwischen etwa 30 und 40 Minuten. Es ist naheliegend, daß bei den Benutzern dieser Haarfarben oder Blondiermittel ein Bedürfnis besteht, diese Einwirkungszeit zu verringern.

Weder die pastenförmigen noch die pulverförmigen Färbe- und/oder Blondiermittel, die heute auf dem Markt sind, können als optimal angesehen werden. Während die Färbe- und/oder Blondierwirkung auf dem Haar als auf die Verbraucherbedürfnisse zugeschnitten bezeichnet werden kann, bestehen doch noch eine Reihe von Nachteilen und Problemen sowohl bei Herstellung als auch bei der Handhabung dieser Mittel.

So verlaufen Colorations- und Blondierprozesse an keratinischen Fasern üblicherweise bei alkalischen pH-Werten, insbesondere zwischen 9,0 und 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Als Alkalisierungsmittel wird üblicherweise Ammoniak eingesetzt, der allerdings für die Anwender den Nachteil des intensiven Geruches und eventueller Reizung aufweist.

In Marktprodukten werden als alternative Alkalisierungsmittel zu Ammoniak beispielsweise Aminomethylpropanol oder Monoethanolamin eingesetzt. Da sie in ihrer Leistung allerdings oft hinter Ammoniak zurückbleiben, werden sie üblicherweise in Abmischung mit Ammoniak eingesetzt. Aber auch hier wird die Leistung des Ammoniaks beeinträchtigt, insbesondere in Bezug auf die Parameter Graubadeckung, Aufhellleistung und Farbergebnis.

Es besteht daher weiterhin ein Bedarf nach Wirkstoffen bzw. Wirkstoffkombinationen für Aufhell- und Färbemittel mit reduziertem Ammoniakgehalt. Insbesondere sollte in ammoniakreduzierten oder gar -freien farbstoff- und/oder wasserstoffperoxidhaltigen Formulierungen eine mit herkömmlichen ammoniakbasierten Mitteln vergleichbare Leistung erzielt werden.

Es wurde nun gefunden, daß sich bestimmte Wirkstoffkombinationen mit besonderem Vorteil in Haarfärbe- und -aufhellungsmittel einarbeiten lassen und zu einer Leistungssteigerung dieser Mittel führen.

Gegenstand der Erfindung ist in einer ersten Ausführungsform ein Mittel zum Aufhellen und/oder Färben von Keratinfasern, insbesondere menschlichen Haaren, enthaltend - jeweils bezogen auf sein Gewicht -
a) 0,001 bis 5 Gew.-% eines oder mehrerer Oxidationsfarbstoffvorprodukte und/oder direktziehender Farbstoffe;
b) 0,1 bis 20 Gew.-% mindestens eines Piperidons und/oder Piperidonderivats dadurch gekennzeichnet, dass es als Piperidonderivat mindestens ein 3-Piperidiniumsalz der Formel (VI) und/oder 4-Piperidiniumsalz der Formel (VII) enthält, 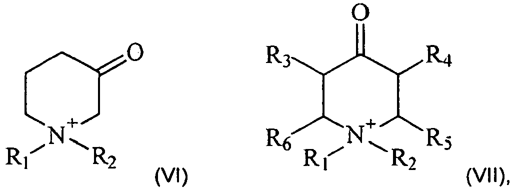 in denen die Reste R¹ bzw. R² bzw. R³ bzw. R⁴ bzw. R⁵ bzw. R⁶ unabhängig voneinander ausgewählt sind aus -H, -CH₃, -CH₂CH₃, -(CH₂)₂CH₂, -CH(CH₃)₂, - (CH₂)₃CH₃, -CH(CH₃)CH₂CH₃, -CH₂CH(CH₃)₂, oder anderen Alkylresten, Alkenylresten, gegebenenfalls substituierten Arylresten und bei denen das Gegenion vorzugsweise ausgewählt ist aus Chlorid, Bromid, Methosulfat, Toluolsulfonat.

Unter Keratinfasern sind dabei Pelze, Wolle, Federn und insbesondere menschliche Haare zu verstehen. Obwohl die erfindungsgemäßen Mittel in erster Linie zum Färben und/oder Aufhellen von Keratinfasern geeignet sind, steht prinzipiell einer Verwendung auch auf anderen Gebieten nichts entgegen.

Die erfindungsgemäßen Mittel enthalten mindestens zwei wesentliche Bestandteile: ein oder mehrere Oxidationsfarbstoffvorprodukte und/oder direktziehende Farbstoffe und mindestens ein Piperidon und/oder Piperidonderivat. Diese werden nachstehend beschrieben.

Vorzugsweise werden die erfindungsgemäßen Mittel als Färbemittel bereitgestellt. Mittel, welche gleichzeitig färbend und aufhellend wirken, werden auch als aufhellende Färbemittel bezeichnet.

Die erfindungsgemäßen Mittel sind Färbemittel, d.h. Mittel zur Veränderung der Farbe keratinischer Fasern. Unter diesen sind insbesondere die sogenannten Oxidationsfärbemittel bevorzugt. Die erfindungsgemäßen Oxidationsfärbemittel enthalten mindestens eine Kuppler- und mindestens eine Entwicklerkomponente. Kuppler- und Entwicklerkomponenten werden auch als Oxidationsfarbstoffvorprodukte bezeichnet. Zudem können die erfindungsgemäßen Oxidationsfärbemittel auch noch direktziehende Farbstoffe als Nuanceure enthalten.

Erfindungsgemäß bevorzugte Mittel zum Färben und/oder Aufhellen keratinischer Fasern sind demnach dadurch gekennzeichnet, daß sie mindestens ein Oxidationsfarbstoffvorprodukt vom Entwicklertyp und/oder Kupplertyp enthalten.

In einer ersten bevorzugten Ausführungsform enthält das Färbemittel weiterhin mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Amino-pyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugte p-Phenylendiamine sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Bevorzugte zweikernige Entwicklerkomponenten sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N, N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.Ganz besonders bevorzugte zweikernige Entwicklerkomponenten sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(ß-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-PyrimidinDerivaten und ihren physiologisch verträglichen Salzen.

Bevorzugte Pyridin-Derivate sind insbesondere die Verbindungen 2,5-Diamino-pyridin, 2-(4'-Methoxyphenyl)amino-3-amino-pyridin, 2,3-Diamino-6-methoxy-pyridin, 2-(ß-Methoxyethyl)amino-3-amino-6-methoxy-pyridin und 3,4-Diamino-pyridin.

Bevorzugte Pyrimidin-Derivate sind insbesondere 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere 4,5-Diamino-1-methylpyrazol, 4,5-Diamino-1-(ß-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(ß-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(ß-hydroxyethyl)amino-1-methylpyrazol.

Unter den Pyrazol-[1,5-a]-pyrimidinen kann man insbesondere nennen: Pyrazol-[1,5-a]-pyrimidin-3,7-diamin; 2,5-Dimethyl-pyrazol-[1,5-a]-pyrimidin-3,7-diamin; Pyrazol-[1,5-a]-pyrimidin-3,5-diamin; 2,7-Dimethyl-pyrazol-[1,5-a]-pyrimidin-3,5-diamin; 3-Aminopyrazol-[1,5-a]-pyrimidin-7-ol; 3-Aminopyrazol-[1,5-a]-pyrimidin-5-ol; 2-(3-Aminopyrazol-[1,5-a]-pyrimidin-7-ylamino)-ethanol; 2-(7-Aminopyrazol-[1,5-a]-pyrimidin-3-ylamino)-ethanol; 2-[(3-Aminopyrazol-[1,5-a]-pyrimidin-7-yl)-(2-hydroxy-ethyl)amino]-ethanol; 2-[(7-Aminopyrazol-[1,5-a]-pyrimidin-3-yl)-(2-hydroxyethyl)amino]-ethanol; 5,6-Dimethylpyrazol-[1,5-a]-pyrimidin-3,7-diamin; 2,6-Dimethylpyrazol-[1,5-a]-pyrimidin-3,7-diamin; 3-Amino-7-dimethylamino-2,5-dimethylpyrazol-[1,5-a]-pyrimidin;sowie ihre physiologisch verträglichen Salze und ihre tautomeren Formen, wenn ein tautomers Gleichgewicht vorhanden ist.

Erfindungsgemäß besonders bevorzugte Oxidationsfärbemittel sind dadurch gekennzeichnet, daß die Entwicklerkomponente ausgewählt ist aus 3-Methyl-1,4-diaminobenzol, 1-(2'-Hydroxyethyl)-2,5-diaminobenzol, 2-(2,5-Diaminophenoxy)-ethanol, N,N-Bis(2'-Hydroxyethyl)-1,4-diamino-benzol, 3-Methyl-4-aminophenol und 2-Methylamino-4-aminophenol, p-Phenylendiamin, 2-(β-Hydroxyethyl)-p-phenylendiamin, N,N-Bis-(ß-hydroxyethyl)-p-phenylendiamin, N,N'-bis-(ß-Hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan, 1,10-Bis-(2',5'-diaminophenyl)-1,4,7,10-tetraoxadecan, p-Aminophenol, 4-Amino-3-fluorphenol, 4-Amino-2-aminomethylphenol, 4-Amino-2-((diethylamino)methyl)phenol, o-Aminophenol, 2-Amino-4-methylphenol, 2-Amino-5- methylphenol, 2-Amino-4-chlorphenol, 2,4,5,6- Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin, 2,5,6-Triaminopyrimidin, 1-(2'-Hydroxy- 5'-aminobenzyl)-imidazolidin-2-on, 4,5-Diamino-1-(2'-hydroxyethyl) pyrazol und *N*-(4-Amino-3-methylphenyl)-*N*-[3-(1*H*-imidazol-1-yl)propyl]amin trihydrochlorid. Bevorzugte erfindungsgemäße Mittel enthalten als Oxidationsfarbstoffvorprodukt mindestens eine Entwicklerkomponente, wobei bevorzugte Entwicklerkomponenten ausgewählt sind aus p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4-Amino-3-methylphenol, Bis-(5-amino-2-hydroxyphenyl)methan, 2,4,5,6-Tetraaminopyrimidin, 2-Hydroxy-4,5,6-triaminopyrimidin, 4,5-Diamino-1-(2-hydroxyethyl)-pyrazol.

Die erfindungsgemäßen Färbemittel können weiterhin mindestens eine Kupplerkomponente enthalten.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxy-pyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)amino-3,4-methylendioxybenzol.

Erfindungsgemäß bevorzugte Mittel zum Färben und/oder Aufhellen keratinischer Fasern sind dadurch gekennzeichnet, daß sie als Oxidationsfarbstoffvorprodukt mindestens eine Kupplerkomponente enthalten, wobei bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

Vorzugsweise werden Kuppler- und Entwicklerkomponenten in einem bestimmten Verhältnis zueinander eingesetzt. Hier sind erfindungsgemäße Oxidationsfärbemittel bevorzugt, die die Kupplerkomponente(n) in einer Menge von 0,01 bis 20 Gew.-%, vorzugsweise von 0,5 bis 5 Gew.-%, und die Entwicklerkomponente(n) in einer Menge von 0,01 bis 20 Gew.-%, vorzugsweise von 0,5 bis 5 Gew.-%, jeweils bezogen auf das gesamte Oxidationsfärbemittel, enthalten.

Zusätzlich können die Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder indophenole, Triphenylmethanfarbstoffe, saure Farbstoffe, basische Farbstoffe, vorzugsweise aus der Gruppe der unter den internationalen Bezeichnungen bzw. Handelsnamen HC Yellow 2, HC Yellow 4, HC Yellow 5, HC Yellow 6, HC Yellow 12, HC Yellow 13, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, Acid Red 92, HC Red BN, Pigment Red 57:1, HC Blue 2, HC Blue 12, Disperse Blue 3, Disperse Red 17, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxyethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol, 3-(2',6'-Diaminopyridyl-3'-azo)-pyridin; 2-((4-(Ethyl(2-hydroxyethyl)-amino)-2-methylphenyl)azo)-5-nitro-1,3-thiazol; NI N-Di(2-hydroxyethyl)-3-methyl-4-((4-nitrophenyl)azo)-anilin; 3-Diethylamino-7-(4-dimethylamino-phenylazo)-5-phenyl-phenaziniumchlorid; 4-(2-Thiazolylazo)-resorcin;4-(((4-Phenylamino)azo)-benzosulfonsäure-natriumsalz; 1 -((3-Aminopropyl)amino)-9,lO-anthracendion; 3',3",4,5,5',5",6,7-Octabromphenolsulfonphtalein;1-((4-Amino-3,5-dimethylphenyl)-(2,6-dichlorphenyl)methylen)-3,5-dimethyl-4-imino-2,5-cyclohexadien-Phosphorsäure(1 :l) (Basic Blue 77); 3',3",5',5"-Tetrabrom-m-kresolsulfonphthalein;2,4-Dinitro-l-naphthol-7-sulfonsäure-Dinatriumsalz;4-[2'-Hydroxy-1'-naphthyl)azo]-benzolsulfonsäure-Natriumsalz;3',6'-Dihydroxy-2',41l51l71-tetraiodospiro-[iso-benzo-furan-l (3H), g1(9H)-xanthenl-3-on-Dinatriumsalz; 6-Hydroxy-5-((2-methoxy-5-methyl-4-sulfophenyl)azo)-2-Naphthalin-sulfonsäure-dinatriumsalz; 2,4-Dinitro-inaphthol-Natriumsalz; 2',4',5',7'-tetrabrom-4,5,6,7,-tetrachlor-3',6'-dihydroxy-Spiro[isobenzofuran-l (3H),9'[9H]xanthen]-3-on-dinatriumsalz;4-(2-Hydroxy-l-naphthylazo)-3-methyl-benzolsulfonsäurenatriumsalz; Basic Orange 31, Basic Blue 6, Basic Blue 7, Basic Blue 9, Basic Blue 26, Basic Blue 41, Basic Blue 99, Basic Brown 4, Basic, Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14 ,Basic Yellow 57, Basic Yellow 87, Basic Red 46.

Erfindungsgemäß bevorzugte Mittel zum Färben und/oder Aufhellen keratinischer Fasern sind dadurch gekennzeichnet, daß sie mindestens einen direktziehenden Farbstoff enthalten, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Acid Yellow 87, Basic Orange 31 und Basic Red 51 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2-Hydroxy-ethyl)amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, .4-Amino-3-nitrophenol, 1-(2-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Verbindungen, die auch unter den Bezeichnungen Basic Yellow 87, Basic Orange 31 und Basic Red 51 bekannt sind, sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als zweiten wesentlichen Inhaltsstoff enthalten die erfindungsgemäßen Mittel mindestens ein Piperidon und/oder mindestens ein Piperidonderivat, wobei es als Piperidonderivat mindestens ein 3-Piperidiniumsalz der Formel (VI) und/oder 4-Piperidiniumsalz der Formel (VII) enthält, 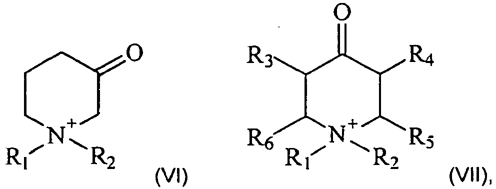 in denen die Reste R¹ bzw. R² bzw. R³ bzw. R⁴ bzw. R⁵ bzw. R⁶ unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, oder anderen Alkylresten, Alkenylresten, gegebenenfalls substituierten Arylresten und bei denen das Gegenion vorzugsweise ausgewählt ist aus Chlorid, Bromid, Methosulfat, Toluolsulfonat.

Piperidone sind Verbindungen, die eine Ketofunktion an einem Sechsring aufweisen, der aus fünf Kohlenstoffatomen und einem Stickkkstoffatom gebildet wird. Je nach Stellung des Stickstoffatomes zur Ketogruppe bezeichnet man den unsubstituierten Grundkörper als 2-Piperidon (Piperidin-2-on, δ-Valerolactam, alpha-Piperidon), 3-Piperidon (Piperidin-3-on, beta-Piperidon) oder 4-Piperidon (Piperidin-4-on, gamma-Piperidon).

Diese drei Piperidone sind erfindungsgemäß einsetzbar. Als zusätzlich zu den Piperidonen oder an ihrer Stelle erfindungsgemäß einsetzbare Piperidonderivate kommen sowohl am C-Gerüst, als auch am Stickstoffatom substituierte Piperidone in Frage, wobei die N-substituierten Piperidonderviate eine große Bedeutung haben, da sich das H-Atom am Stickstoff leicht austauschen läßt. Unter diesen Piperidonderviaten haben sich erfindungsgemäß folgende als besonders bevorzugt erwiesen:1-Methyl-Piperidin-4-on (N-Methyl-4-piperidon),1-Methyl-Piperidin-3-on (N-Methyl-3-piperidon),1-Methyl-Piperidin-2-on (N-Methyl-2-piperidon), 1-Ethyl-Piperidin-4-on (N-Ethyl-4-piperidon), 1-Ethyl-Piperidin-3-on (N-Ethyl-3-piperidon), 1-Ethyl-Piperidin-2-on (N-Ethyl-2-piperidon), 1-Propyl-Piperidin-4-on (N-Propyl-4-piperidon), 1-Propyl-Piperidin-3-on (N-Propyl-3-piperidon), 1-Propyl-Piperidin-2-on (N-Propyl-2-piperidon), 1-Isopropyl-Piperidin-4-on (N-Isopropyl-4-piperidon), 1-Isopropyl-Piperidin-3-on (N-Isopropyl-3-piperidon), 1-Isopropyl-Piperidin-2-on (N-Isopropyl-2-piperidon), 1-Butyl-Piperidin-4-on (N-Butyl-4-piperidon), 1-Butyl-Piperidin-3-on (N-Butyl-3-piperidon), 1-Butyl-Piperidin-2-on (N-Butyl-2-piperidon), 1-Pentyl-Piperidin-4-on (N-Pentyl-4-piperidon), 1-Pentyl-Piperidin-3-on (N-Pentyl-3-piperidon), 1-Pentyl-Piperidin-2-on (N-Pentyl-2-piperidon), 1-Hexyl-Piperidin-4-on (N-Hexyl-4-piperidon), 1-Hexyl-Piperidin-3-on (N-Hexyl-3-piperidon), 1-Hexyl-Piperidin-2-on (N-Hexyl-2-piperidon), 1-Heptyl-Piperidin-4-on (N-Heptyl-4-piperidon), 1-Heptyl-Piperidin-3-on (N-Heptyl-3-piperidon), 1-Heptyl-Piperidin-2-on (N-Heptyl-2-piperidon), 1-Octyl-Piperidin-4-on (N-Octyl-4-piperidon), 1-Octyl-Piperidin-3-on (N-Octyl-3-piperidon), 1-Octyl-Piperidin-2-on (N-Octyl-2-piperidon), 1-Nonyl-Piperidin-4-on (N-Nonyl-4-piperidon), 1-Nonyl-Piperidin-3-on (N-Nonyl-3-piperidon), 1-Nonyl-Piperidin-2-on (N-Nonyl-2-piperidon), 1-Decyl-Piperidin-4-on (N-Decyl-4-piperidon), 1-Decyl-Piperidin-3-on (N-Decyl-3-piperidon), 1-Decyl-Piperidin-2-on (N-Decyl-2-piperidon).

Erfindungsgemäß besonders bevorzugte Mittel enthalten 0,1 bis 17,5 Gew.-%, vorzugsweise 0,5 bis 15 Gew.-%, besonders bevorzugt 1 bis 12,5 Gew.-% und insbesondere 2,5 bis 10 Gew.-% mindestens einer Verbindung aus der Gruppe
- 2-Piperidon und/oder
- der Derivate von 2-Piperidon, insbesondere der 2-Piperidiniumsalze und/oder
- 3-Piperidon und/oder
- der Derivate von 3-Piperidon, insbesondere der 3-Piperidiniumsalze und/oder
- 4-Piperidon und/oder
- der Derivate von 4-Piperidon, insbesondere der 4-Piperidiniumsalze

ErfindungsgemäßMittel sind dadurch gekennzeichnet, daß sie 3-Piperidiniumsalze der Formel (VI) und/oder 4-Piperidiniumsalze der Formel (VII) enthalten 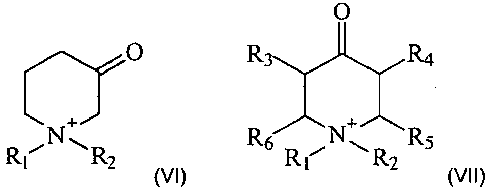 in denen die Reste R¹ bzw. R² bzw. R³ bzw. R⁴ bzw. R⁵ bzw. R⁶ unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, - CH₂CH(CH₃)₂, oder anderen Alkylresten, Alkenylresten, gegebenenfalls substituierten Arylresten und bei denen das Gegenion vorzugsweise ausgewählt ist aus Chlorid, Bromid, Methosulfat, Toluolsulfonat. Vorzugsweise können die Reste bzw. R³ bzw. R⁴ bzw. R⁵ bzw. R⁶ ein Wasserstoffatom sein. Unter diesen Piperidiniumsalzen haben sich erfindungsgemäß folgende als besonders bervozugt erwiesen:3-oxo-Piperidinium-Chlorid (R¹ = R² = H in Formel VI), 4-oxo-Piperidinium-Chlorid (R¹= R² = H in Formel VII), 3-oxo-Piperidinium-Bromid (R¹ = R² = H in Formel VI), 4-oxo-Piperidinium-Bromid (R¹ = R² = H in Formel VII), 3-oxo-Piperidinium-Methosulfat (R¹ = R² = H in Formel VI), 4-oxo-Piperidinium-Methosulfat (R¹ = R² = H in Formel VII), 3-oxo-Piperidinium-Tosylat (R¹ = R² = H in Formel VI), 4-oxo-Piperidinium-Tosylat (R¹ = R² = H in Formel VII), N-Methyl-3-oxo-piperidinium-Chlorid (R¹= -CH₃, R² = H in Formel VI), N-Methyl-4-oxo-piperidinium-Chlorid (R¹ = - CH₃, R² = H in Formel VII), N-Methyl-3-oxo-piperidinium-Bromid (R¹= -CH₃, R² = H in Formel VI), N-Methyl-4-oxo-piperidinium-Bromid (R¹ = -CH₃, R² = H in Formel VII), N-Methyl-3-oxo-piperidinium-Methosulfat (R¹= -CH₃, R² = H in Formel VI), N-Methyl-4-oxo-piperidinium-Methosulfat (R¹ = -CH₃, R² = H in Formel VII), N-Methyl-3-oxo-piperidinium-Tosylat (R¹= -CH₃, R² = H in Formel VI), N-Methyl-4-oxo-piperidinium-Tosylat (R¹= -CH₃, R² = H in Formel VII), N-Ethyl-3-oxo-piperidinium-Chlorid (R¹= -CH₂CH₃, R² = H in Formel VI), N-Ethyl-4-oxo-piperidinium-Chlorid (R¹= -CH₂CH₃, R² = H in Formel VII), N-Ethyl-3-oxo-piperidinium-Bromid (R¹= -CH₂CH₃, R² = H in Formel VI), N-Ethyl-4-oxo-piperidinium-Bromid (R¹= -CH₂CH₃, R² = H in Formel VII), N-Ethyl-3-oxo-piperidinium-Methosulfat (R¹= -CH₂CH₃, R² = H in Formel VI), N-Ethyl-4-oxo-piperidinium-Methosulfat (R¹= -CH₂CH₃, R² = H in Formel VII), N-Ethyl-3-oxo-piperidinium-Tosylat (R¹= -CH₂CH₃, R² = H in Formel VI), N-Ethyl-4-oxo-piperidinium-Tosylat (R¹= -CH₂CH₃, R² = H in Formel VII), N-Propyl-3-oxo-piperidinium-Chlorid (R¹= -CH₂CH₂CH₃, R² = H in Formel VI), N-Propyl-4-oxo-piperidinium-Chlorid (R¹ = -CH₂CH₂CH₃, R² = H in Formel VII), N-Propyl-3-oxo-piperidinium-Bromid (R¹= -CH₂CH₂CH₃, R² = H in Formel VI), N-Propyl-4-oxo-piperidinium-Bromid (R¹= -CH₂CH₂CH₃, R² = H in Formel VII), N-Propyl-3-oxo-piperidinium-Methosulfat (R¹ = - CH₂CH₂CH₃, R² = H in Formel VI), N-Propyl-4-oxo-piperidinium-Methosulfat (R¹= -CH₂CH₂CH₃, R² = H in Formel VII), N-Propyl-3-oxo-piperidinium-Tosylat (R¹ = -CH₂CH₂CH₃, R² = H in Formel VI), N-Propyl-4-oxo-piperidinium-Tosylat (R¹= -CH₂CH₂CH₃, R² = H in Formel VII), N-Isopropyl-3-oxo-piperidinium-Chlorid (R¹ = -CH(CH₃)₂, R² = H in Formel VI), N-Isopropyl-4-oxo-piperidinium-Chlorid (R¹ = -CH(CH₃)₂, R² = H in Formel VII), N-Isopropyl-3-oxo-piperidinium-Bromid (R¹ = - CH(CH₃)₂R² = H in Formel VI), N-Isopropyl-4-oxo-piperidinium-Bromid (R¹= -CH(CH₃)₂, R² = H in Formel VII), N-Isopropyl-3-oxo-piperidinium-Methosulfat (R¹= -CH(CH₃)₂, R² = H in Formel VI), N-Isopropyl-4-oxo-piperidinium-Methosulfat (R¹ -CH(CH₃)₂, R² = H in Formel VII), N-Isopropyl-3-oxo-piperidinium-Tosylat (R¹= -CH(CH₃)₂, R² = H in Formel VI), N-Isopropyl-4-oxo-piperidinium-Tosylat (R¹= -CH(CH₃)₂, R² = H in Formel VII), N-Butyl-3-oxo-piperidinium-Chlorid (R¹ = - (CH₂)₃CH₃, R² = H in Formel VI), N-Butyl-4-oxo-piperidinium-Chlorid (R¹= -(CH₂)₃CH₃, R² = H in Formel VII), N-Butyl-3-oxo-piperidinium-Bromid (R¹= -(CH₂)₃CH₃, R² = H in Formel VI), N-Butyl-4-oxo-piperidinium-Bromid (R¹ = -(CH₂)₃CH₃, R² = H in Formel VII), N-Butyl-3-oxo-piperidinium-Methosulfat (R¹ = -(CH₂)₃CH₃, R² = H in Formel VI), N-Butyl-4-oxo-piperidinium-Methosulfat (R¹ = -(CH₂)₃CH₃, R² = H in Formel VII), N-Butyl-3-oxo-piperidinium-Tosylat (R¹ = -(CH₂)₃CH₃, R² = H in Formel VI), N-Butyl-4-oxo-piperidinium-Tosylat (R¹= -(CH₂)₃CH₃, R² = H in Formel VII), N-Isobutyl-3-oxo-piperidinium-Chlorid (R¹ = -CH₂CH(CH₃)₂, R² = H in Formel VI), N-Isobutyl-4-oxo-piperidinium-Chlorid (R¹ = -CH₂CH(CH₃)₂, R² = H in Formel VII), N-Isobutyl-3-oxo-piperidinium-Bromid (R¹= -CH₂CH(CH₃)₂, R² = H in Formel VI), N-Isobutyl-4-oxo-piperidinium-Bromid (R¹ = - CH₂CH(CH₃)₂, R² = H in Formel VII), N-Isobutyl-3-oxo-piperidinium-Methosulfat (R¹ = - CH₂CH(CH₃)₂, R² = H in Formel VI), N-Isobutyl-4-oxo-piperidinium-Methosulfat (R¹ = - CH₂CH(CH₃)₂, R² = H in Formel VII), N-Isobutyl-3-oxo-piperidinium-Tosylat (R¹ = -CH₂CH(CH₃)₂, R² = H in Formel VI), N-Isobutyl-4-oxo-piperidinium-Tosylat (R¹= -CH₂CH(CH₃)₂, R² = H in Formel VII), N-sec-Butyl-3-oxo-piperidinium-Chlorid (R¹ = -CH₂CH(CH₃)₂, R² = H in Formel VI), N-sec-Butyl-4-oxo-piperidinium-Chlorid (R¹ = -CH₂CH(CH₃)₂, R² = H in Formel VII), N-sec-Butyl-3-oxo-piperidinium-Bromid (R¹ = -CH₂CH(CH₃)₂, R² = H in Formel VI), N-sec-Butyl-4-oxo-piperidinium-Bromid (R¹ = -CH₂CH(CH₃)₂, R² = H in Formel VII), N-sec-Butyl-3-oxo-piperidinium-Methosulfat (R¹ = -CH₂CH(CH₃)₂, R² = H in Formel VI), N-*sec*-Butyl-4-oxo-piperidinium-Methosulfat (R¹ = - CH₂CH(CH₃)₂, R² = H in Formel VII), N-sec-Butyl-3-oxo-piperidinium-Tosylat (R¹ = -CH₂CH(CH₃)₂, R² = H in Formel VI), N-*sec*-Butyl-4-oxo-piperidinium-Tosylat (R¹= -CH₂CH(CH₃)₂, R² = H in Formel VII), N,N-Dimethyl-3-oxo-piperidinium-Chlorid (R¹ = R² = -CH₃ in Formel VI), N,N-Dimethyl-4-oxo-piperidinium-Chlorid (R¹= R² = -CH₃ in Formel VII), N,N-Dimethyl-3-oxo-piperidinium-Bromid (R¹ = R² = -CH₃ in Formel VI), N,N-Dimethyl-4-oxo-piperidinium-Bromid (R¹ = R² = -CH₃ in Formel VII), N,N-Dimethyl-3-oxo-piperidinium-Methosulfat (R¹ = R² = -CH₃ in Formel VI), N,N-Dimethyl-4-oxo-piperidinium-Methosalufat (R¹ = R² = -CH₃ in Formel VII), N,N-Dimethyl-3-oxo-piperidinium-Tosylat (R¹= R² = -CH₃ in Formel VI), N,N-Dimethyl-4-oxo-piperidinium-Tosylat (R¹ = R² = -CH₃ in Formel VII), N,N-Diethyl-3-oxo-piperidinium-Chlorid (R¹= -CH₂CH₃, R² = H in Formel VI), N,N-Diethyl-4-oxo-piperidinium-Chlorid (R¹= -CH₂CH₃, R² = H in Formel VII), N,N-Diethyl-3-oxo-piperidinium-Bromid (R¹= -CH₂CH₃, R² = H in Formel VI), N,N-Diethyl-4-oxo-piperidinium-Bromid (R¹= -CH₂CH₃, R² = H in Formel VII), N,N-Diethyl-3-oxo-piperidinium-Methosulfat (R¹ = -CH₂CH₃, R² = H in Formel VI), N,N-Diethyl-4-oxo-piperidinium-Methosulfat (R¹ = -CH₂CH₃, R² = H in Formel VII), N,N-Diethyl-3-oxo-piperidinium-Tosylat (R¹ = -CH₂CH₃, R² = H in Formel VI), N,N-Diethyl-4-oxo-piperidinium-Tosylat (R¹= -CH₂CH₃, R² = H in Formel VII), N,N-Dipropyl-3-oxo-piperidinium-Chlorid (R¹ = R² = -CH₂CH₂CH₃ in Formel VI), N,N-Dipropyl-4-oxo-piperidinium-Chlorid (R¹ = R² = -CH₂CH₂CH₃ in Formel VII), N,N-Dipropyl-3-oxo-piperidinium-Bromid (R¹ = R² = -CH₂CH₂CH₃ in Formel VI), N,N-Dipropyl-4-oxo-piperidinium-Bromid (R¹ = R² = -CH₂CH₂CH₃ in Formel VII), N,N-Dipropyl-3-oxo-piperidinium-Methosulfat (R¹ = R² = -CH₂CH₂CH₃ in Formel VI), N,N-Dipropyl-4-oxo-piperidinium-Methosulfat (R¹ = R² = -CH₂CH₂CH₃ in Formel VII), N,N-Dipropyl-3-oxo-piperidinium-Tosylat (R¹ = R² = -CH₂CH₂CH₃ in Formel VI), N,N-Dipropyl-4-oxo-piperidinium-Tosylat (R¹ = R² = -CH₂CH₂CH₃ in Formel VII), N,N-Diisopropyl-3-oxo-piperidinium-Chlorid (R¹ = R² = -CH(CH₃)₂ in Formel VI), N,N-Diisopropyl-4-oxo-piperidinium-Chlorid (R¹ = R² = -CH(CH₃)₂ in Formel VII), N,N-Diisopropyl-3-oxo-piperidinium-Bromid (R¹ = R² = -CH(CH₃)₂ in Formel VI), N,N-Diisopropyl-4-oxo-piperidinium-Bromid (R¹ = R² = -CH(CH₃)₂ in Formel VII), N,N-Diisopropyl-3-oxo-piperidinium-Methosulfat (R¹ = R² = -CH(CH₃)₂ in Formel VI), N,N-Diisopropyl-4-oxo-piperidinium-Methosulfat (R¹ = R² = -CH(CH₃)₂ in Formel VII), N,N-Diisopropyl-3-oxo-piperidinium-Tosylat (R¹ = R² = -CH(CH₃)₂ in Formel VI), N,N-Diisopropyl-4-oxo-piperidinium-Tosylat (R¹ = R² = -CH(CH₃)₂ in Formel VII), N,N-Dibutyl-3-oxo-piperidinium-Chlorid (R¹ = R² = -(CH₂)₃CH₃ in Formel VI), N,N-Dibutyl-4-oxo-piperidinium-Chlorid (R¹ = R² = - (CH₂)₃CH₃ in Formel VII), N,N-Dibutyl-3-oxo-piperidinium-Bromid (R¹= R² = -(CH₂)₃CH₃ in Formel VI), N,N-Dibutyl-4-oxo-piperidinium-Bromid (R¹ = R² = -(CH₂)₃CH₃ in Formel VII), N,N-Dibutyl-3-oxo-piperidinium-Methosulfat (R¹ = R² = -(CH₂)₃CH₃ in Formel VI), N,N-Dibutyl-4-oxo-piperidinium-Methosulfat (R¹ = R² = -(CH₂)₃CH₃ in Formel VII), N,N-Dibutyl-3-oxo-piperidinium-Tosylat (R¹ = R² = -(CH₂)₃CH₃ in Formel VI), N,N-Dibutyl-4-oxo-piperidinium-Tosylat (R¹ = R² = - (CH₂)₃CH₃ in Formel VII), N,N-Diisobutyl-3-oxo-piperidinium-Chlorid (R¹= -CH₂CH (CH₃)₂, R² = H in Formel VI), N,N-Diisobutyl-4-oxo-piperidinium-Chlorid (R¹ = -CH₂CH (CH₃)₂, R² = H in Formel VII), N,N-Diisobutyl-3-oxo-piperidinium-Bromid (R¹= -CH₂CH (CH₃)₂, R² = H in Formel VI), N,N-Diisobutyl-4-oxo-piperidinium-Bromid (R¹= -CH₂CH (CH₃)₂, R² = H in Formel VII), N,N-Diisobutyl-3-oxo-piperidinium-Methosulfat (R¹= -CH₂CH (CH₃)₂, R² = H in Formel VI), N,N-Diisobutyl-4-oxo-piperidinium-Methosulfat (R¹= -CH₂CH (CH₃)₂, R² = H in Formel VII), N,N-Diisobutyl-3-oxo-piperidinium-Tosylat (R¹ = -CH₂CH (CH₃)₂, R² = H in Formel VI), N,N-Diisobutyl-4-oxo-piperidinium-Tosylat (R¹ = -CH₂CH (CH₃)₂, R² = H in Formel VII), N,N-Di(sec-Butyl)-3-oxo-piperidinium-Chlorid (R¹ = R² = -CH₂CH(CH₃)₂ in Formel VI), N,N-Di(sec-Butyl)-4-oxo-piperidinium-Chlorid (R¹ = R² = -CH₂CH(CH₃)₂ in Formel VII), N,N-Di(sec-Butyl)-3-oxo-piperidinium-Bromid (R¹ = R² = -CH₂CH(CH₃)₂ in Formel VI), N,N-Di(sec-Butyl)-4-oxo-piperidinium-Bromid (R¹ = R² = -CH₂CH(CH₃)₂ in Formel VII), N,N-Di(sec-Butyl)-3-oxo-piperidinium-Methosulfat (R¹ = R² = -CH₂CH(CH₃)₂ in Formel VI), N,N-Di(sec-Butyl)-4-oxo-piperidinium-Methosulfat (R¹ = R² = -CH₂CH(CH₃)₂ in Formel VII), N,N-Di(sec-Butyl)-3-oxo-piperidinium-Tosylat (R¹ = R² = -CH₂CH(CH₃)₂ in Formel VI), N,N-Di(sec-Butyl)-4-oxo-piperidinium-Tosylat (R¹ = R² = -CH₂CH(CH₃)₂ in Formel VII), N,N-3,5-Tetramethyl-4-oxo-piperidinium-lodid (R¹ = R² = R³ = R⁴ = -CH₃ in Formel VII), N,N-2,6-Tetramethyl-4-oxo-piperidinium-lodid (R¹ = R² = R⁵ = R⁶ = -CH₃ in Formel VII).

Als weiteren Inhaltsstoff können die erfindungsgemäßen Mittel mit besonderem Vorzug ein oder mehrere Imidazolverbindungen enthalten. Erfindungsgemäß besonders bevorzugte Mittel sind dadurch gekennzeichnet, daß sie zusätzlich 0,1 bis 15 Gew.-%, vorzugsweise 0,5 bis 12,5 Gew.-%, besonders bevorzugt 1 bis 10 Gew.-% und insbesondere 3 bis 8 Gew.-% mindestens einer Imidazolverbindung gemäß Formel (VIII) und/oder deren physiologisch verträglichen Salzen 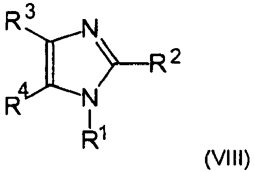 enthalten, worin
- R¹: steht für ein Wasserstoffatom, eine gegebenenfalls substituierte Arylgruppe oder eine (C₁-C₆)-Alkylgruppe,
- R²: steht für ein Wasserstoffatom, eine Carboxaldehydgruppe, eine (C₁-C₆)-Alkylgruppe oder eine Nitrogruppe,
- R³: steht für ein Wasserstoffatom, eine Carboxy-(C₁-C₆)-alkylgruppe, eine Amino-(C₁-C₆)-alkylgruppe, eine Carboxylgruppe, eine Carboxaldehydgruppe, eine (C₁-C₆)-Alkylgruppe, eine Nitrogruppe, eine 2-Amino-3-hydroxypropylgruppe oder eine Gruppe -CH₂-CH(NH₂)-COOH,
- R⁴: steht für ein Wasserstoffatom, eine Carboxaldehydgruppe oder eine Carboxylgruppe.

Vorzugsweise wird/werden die Imidazolverbindung(en) der Formel (VIII) innerhalb engerer Mengenbereiche eingesetzt. Hier sind erfindungsgemäße Mittel bevorzugt, die mindestens eine Imidazolverbindung gemäß Formel (VIII) und/oder deren physiologisch verträgliche Salze in Mengen von 0,5 bis 15 Gew.-%, vorzugsweise von 2,5 bis 12,5 Gew.-%, besonders bevorzugt von 3 bis 10 Gew.-% und insbesondere von 4 bis 8 Gew.-% enthalten.

Bevorzugt werden die Imidazolverbindungen gemäß Formel I ausgewählt aus mindestens einem Vertreter aus einer Gruppe, die gebildet wird, aus Histamin, D-Histidin, L-Histidin, DL-Histidin, D-Histidinol, L-Histidinol, DL-Histidinol, Imidazol, Imidazol-4-essigsäure, Imidazol-4-carbonsäure, Imidazol-4,5-dicarbonsäure, Imidazol-2-carboxaldehyd, Imidazol-4-carboxaldehyd, Imidazol-5-carboxaldehyd, 2-Nitroimidazol, 4-Nitroimidazol, 4-Methylimidazol-5-carboxaldehyd, N-Methylimidazol-2-carboxaldehyd, 4-Methylimidazol, 2-Methylimidazol, N-Methylimidazol, N-(4-Aminophenyl)-imidazol, sowie den physiologisch verträglichen Salzen der vorgenannten Verbindungen. Erfindungsgemäß besonders bevorzugt wird Imidazol eingesetzt.

Die erfindungsgemäßen Mittel enthalten gegebenenfalls Ammoniak (berechnet als 100 %iges NH₃), wobei dessen Menge auf maximal 3 Gew.-% - bezogen auf das gesamte Mittel bzw. bei Mehrkomponentenmitteln bezogen auf die anwendungsfertige Mischung - begrenzt ist. Bevorzugte erfindungsgemäße Mittel bzw. Anwendungsmischungen enthalten maximal 1,8 Gew.-% Ammoniak, weiter bevorzugt maximal 1,5 Gew.-% Ammoniak, noch weiter bevorzugt maximal 1,25 Gew.-% Ammoniak, besonders bevorzugt maximal 1,0 Gew.-% Ammoniak und insbesondere maximal 0,5 Gew.-% Ammoniak. In einer weiter bevorzugten Ausführungsform der vorliegenden Erfindung sind die Mittel ammoniakfrei.

Wie bereits erwähnt, können die erfindungsgemäßen Mittel auch direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden.

Ein üblicher Weg besteht daher darin, ein erfindungsgemäßes Mittel A direkt vor der Anwendung mit einer Oxidationsmittelzubereitung B zu einer Anwendungsmischung zu vermischen.

Ein weiterer Gegenstand der vorliegenden Erfindung ist daher ein Mittel zum Färben und/oder Aufhellen keratinischer Fasern, insbesondere menschlicher Haare, welches unmittelbar vor dem Aufbringen auf das Haar aus einer fließfähigen Zubereitung A, einer Oxidationsmittelzubereitung B, enthaltend mindestens ein Oxidationsmittel, ausgewählt aus Wasserstoffperoxid und dessen Anlagerungsverbindungen an feste Träger und einer Zubereitung C, die miteinander zu einem Färbe- und/oder Aufhellansatz vermischt werden, erhalten wird, wobei die Zusammensetzung A und/oder C eine erfindungsgemäße Zusammensetzung ist, oder die Zusammensetzung A Oxidationsfarbstoffvorprodukt(e) und die Zusammensetzung C Piperidon(e) und/oder Piperidonderivat(e) enthält (oder vice versa).

Die Mischung der Zubereitungen A, B und C vor der Anwendung führt zu einer Anwendungsmischung, die Oxidationsfarbstoffvorprodukt(e) und Piperidon(e) bzw. Piperidonderivat(e) enthält. Bevorzugte Anwendungsmischungen genügen darüber hinaus der ersten Ausführungsform der vorliegenden Erfindung, d.h. besonders bevorzugte Mittel der zweiten Ausführungsform sind so konfektioniert und/oder werden in einem derartigen Verhältnis miteinander vermischt, daß die Anwendungmischung die genannten Mengen an Oxidationsfarbstoffvorprodukt(en) und Piperidon(en) und/oder Piperidonderivat(en) enthält.

Eine Oxidationsmittelzubereitung B enthält mindestens ein Oxidationsmittel, ausgewählt aus Wasserstoffperoxid und dessen Anlagerungsverbindungen an feste Träger. Als erfindungsgemäßes Oxidationsmittel wird bevorzugt Wasserstoffperoxid selbst verwendet. Das Wasserstoffperoxid wird als Lösung oder in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon n H₂O₂ (n ist eine positive ganze Zahl größer 0), Harnstoffperoxid und Melaminperoxid, eingesetzt. Erfindungsgemäß ganz besonders bevorzugt sind wässrige Wasserstoffperoxid-Lösungen. Die Konzentration einer Wasserstoffperoxid-Lösung wird einerseits von den gesetzlichen Vorgaben und andererseits von dem gewünschten Effekt bestimmt; vorzugsweise werden 6- bis 12-prozentige Lösungen in Wasser verwendet. Erfindungsgemäß bevorzugte Mittel zum Färben und/oder Aufhellen keratinischer Fasern sind dadurch gekennzeichnet, daß die Oxidationsmittelzubereitung B - bezogen auf ihr Gewicht - 0,5 bis 10 Gew.-%, vorzugsweise 0,75 bis 9,0 Gew.-%, besonders bevorzugt 1 bis 8,0 Gew.-%, weiter bevorzugt 1,25 bis 7,0 Gew.-% und insbesondere 1,5 bis 6,0 Gew.-% Wasserstoffperoxid (berechnet als 100 %iges H₂O₂) enthält.

Die Oxidationsmittelzubereitung B ist vorzugsweise eine wäßrige, fließfähige Oxidationsmittelzubereitung. Demnach sind bevorzugte erfindungsgemäße Mittel zum Färben und/oder Aufhellen keratinischer Fasern dadurch gekennzeichnet, daß die fließfähige Oxidationsmittelzubereitung B - bezogen auf ihr Gewicht - 40 bis 90 Gew.-%, vorzugsweise 50 bis 85 Gew.-%, besonders bevorzugt 55 bis 80 Gew.-%, weiter bevorzugt 60 bis 77,5 Gew.-% und insbesondere 65 bis 75 Gew.-% Wasser enthält.

Bezogen auf die Anwendungsmischung oder bezogen auf Einkomponentenmittel sind erfindungsgemäße Mittel bevorzugt, die 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthalten.

Vorzugsweise wird der fließfähigen Oxidationsmittelzubereitung B ein Emulgator bzw. ein Tensid zugesetzt, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind. Diese Stoffe werden weiter unten ausführlich beschrieben.

Die Oxidationsmittelzubereitung kann weitere Oxidationsmittel enthalten, die auch als "Booster" bezeichnet werden. Die Auswahl dieser weiteren Peroxoverbindung unterliegt prinzipiell keinen Beschränkungen; übliche, dem Fachmann bekannte Peroxoverbindungen sind beispielsweise Ammoniumperoxidisulfat, Kaliumperoxidisulfat, Natriumperoxidisulfat, Ammoniumpersulfat, Kaliumpersulfat, Natriumpersulfat, Percarbonate wie Magnesiumpercarbonat, Peroxide wie Bariumperoxid sowie Perborate, Harnstoffperoxid und Melaminperoxid. Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Kombinationen aus mindestens zwei Peroxidisulfaten. Hier sind erfindungsgemäße Mittel zum Färben und/oder Aufhellen keratinischer Fasern bevorzugt, die zusätzlich 0,01 bis 2 Gew.-% mindestens einer festen Peroxoverbindung, die ausgewählt ist aus Ammonium-, Alkalimetall- und Erdalkalimetallpersulfaten, -peroxomonosulfaten und -peroxidisulfaten enthalten, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei besonders bevorzugte Mittel mindestens zwei verschiedene Peroxidisulfate enthalten.

Weiterhin besonders bevorzugt sind Persulfate, insbesondere das als Carosche Salz bezeichnete Gemisch aus Kaliumperoxosulfat, Kaliumhydrogensulfat und Kaliumsulfat.

Im Falle vonm Mehrkomponentenmitteln enthält vorzugsweise die fließfähige Oxidationsmittelzubereitung B zusätzlich mindestens eine Peroxoverbindung, die vorzugsweise ausgewählt ist aus Ammonium- und Alkalimetall-persulfaten und -peroxidisulfaten, wobei bevorzugte Mittel mindestens 2 verschiedene Peroxidisulfate enthalten.

Die erfindungsgemäßen wasserfreien Zusammensetzungen können zusätzlich mindestens einen weiteren Bleichverstärker, der von den anorganischen Persalzen verschieden ist, enthalten.

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Zur weiteren Steigerung der Aufhellleistung können erfindungsgemäße wasserfreie Mittel zusätzlich als Bleichverstärker mindestens eine gegebenenfalls hydratisierte SiO₂-Verbindung enthalten. Obwohl bereits geringe Mengen der gegebenenfalls hydratisierten SiO₂-Verbindungen die Aufhellleistung erhöhen, kann es erfindungsgemäß bevorzugt sein, die gegebenenfalls hydratisierten SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße wasserfreie Zusammensetzung, einzusetzen. Die Mengenangaben geben dabei jeweils den Gehalt der SiO₂-Verbindungen (ohne deren Wasseranteil) in den Mitteln wieder.

Hinsichtlich der gegebenenfalls hydratisierten SiO₂-Verbindungen unterliegt die vorliegende Erfindung prinzipiell keinen Beschränkungen. Bevorzugt sind Kieselsäuren, deren Oligomeren und Polymeren sowie deren Salze. Bevorzugte Salze sind die Alkalisalze, insbesondere die Kalium und Natriumsalze. Die Natriumsalze sind ganz besonders bevorzugt.

Die gegebenenfalls hydratisierten SiO₂-Verbindungen können in verschiedenen Formen vorliegen. Erfindungsgemäß bevorzugt werden die SiO₂-Verbindungen in Form von Kieselgelen (Silicagel) oder besonders bevorzugt als Wasserglas eingesetzt. Diese SiO₂-Verbindungen können teilweise in wäßriger Lösung vorliegen.

Erfindungsgemäß ganz besonders bevorzugt sind Wassergläser, die aus einem Silikat der Formel (SiO₂)ₙ(Na₂O)ₘ(K₂O)ₚ gebildet werden, wobei n steht für eine positive rationale Zahl und m und p stehen unabhängig voneinander für eine positive rationale Zahl oder für 0, mit den Maßgaben, daß mindestens einer der Parameter m oder p von 0 verschieden ist und das Verhältnis zwischen n und der Summe aus m und p zwischen 1:4 und 4:1 liegt.

Neben den durch die Summenformel beschriebenen Komponenten können die Wassergläser in geringen Mengen noch weitere Zusatzstoffe, wie beispielsweise Phosphate oder Magnesiumsalze, enthalten.

Erfindungsgemäß bevorzugte Mittel sind dadurch gekennzeichnet, daß sie zusätzlich 0,05 bis 15 Gew.-%, besonders bevorzugt 0,15 bis 10 Gew.-% und insbesondere 0,2 bis 5 Gew.-% mindestens einer gegebenenfalls hydratisierte SiO₂-Verbindung enthalten, wobei bevorzugte Mittel Wassergläser enthalten, die aus einem Silikat der Formel (SiO₂)ₙ(Na₂O)ₘ(K₂O)ₚ gebildet werden, worin n steht für eine positive rationale Zahl und m und p unabhängig voneinander für eine positive rationale Zahl oder für 0 stehen, mit den Maßgaben, daß mindestens einer der Parameter m oder p von 0 verschieden ist und das Verhältnis zwischen n und der Summe aus m und p zwischen 1:4 und 4:1 liegt.

Zusätzlich zu den genannten Inhaltsstoffen hat sich der Einsatz von Carbonaten und Carbonatanaloga in den erfindungsgemäßen Mitteln als bevorzugt herausgestellt. Erfindungsgemäß bevorzugte Mittel enthalten zusätzlich 0,1 bis 25 Gew.%, vorzugsweise 0,5 bis 20 Gew.%, besonders bevorzugt 1 bis 15 Gew.% und insbesondere 1,5 bis 10 Gew.% mindestens eines Stoffes aus den Gruppen der
- Carbonate und/oder
- Monoalkylcarbonate (Kohlensäuremonoester) und/oder
- Kohlensäuremonoamide und/oder
- Silylcarbonate und/oder
- Silylcarbamate.

Als Carbonate bieten sich insbesondere Ammonium-, Alkalimetall- und Erdalkalimetallcarbonate und -hydrogencarbonate an. Besonders bevorzugt sind beispielsweise Natriumhydrogencarbonat, Ammoniumhydrogencarbonat, Natriumcarbonat, Ammoniumcarbonat und Ammoniumcarbamat.

Zusätzlich zu Carbonaten oder an deren Stelle können die erfindungsgenmäßen Mittel vorzugsweise auch mindestens einen Kohlensäuremonoester und/oder mindestens ein Kohlensäuremonoamid enthalten. Diese Substanzen werden nachfolgend detailliert beschrieben.

Erfindungsgemäß bevorzugte Mittel sind dadurch gekennzeichnet, daß sie mindestens einen Kohlensäuremonoester der Formel (IX): R-O-C(O)-O-H (IX) enthalten, in der R für einen gesättigten oder ungesättigten, geradkettigen, verzweigten, oder cyclischen, substituierten oder unsubstituierten Kohlenwasserstoffrest, oder eine substituierte oder unsubstiuierte Arylgruppe bzw. einen substituierten oder unsubstituierten Heterocyclus steht.

Erfindungsgemäß besonders bevorzugte Mittel sind dadurch gekennzeichnet, daß der Rest R in Formel (IX) ausgewählt ist aus Methyl-, Ethyl-, n-Propyl-, iso-Propyl-, n-Butyl-, iso-Butyl-, *tert-*Butyl- sowie Hydroxymethyl- und Hydroxyethyl-Resten.

Alternativ zum Kohlensäuremonoester oder in Verbindung mit ihm können die erfindungsgemäßen Mittel Kohlensäuremonoamide enthalten. Hier sind erfindungsgemäß bevorzugte Mittel dadurch gekennzeichnet, daß sie mindestens ein Kohlensäuremonoamid der Formel (X): R-NH-C(O)-O-H (X) enthält, in der R für einen gesättigten oder ungesättigten, geradkettigen, verzweigten, oder cyclischen, substituierten oder unsubstituierten Kohlenwasserstoffrest, oder eine substituierte oder unsubstiuierte Arylgruppe bzw. einen substituierten oder unsubstituierten Heterocyclus steht.

Erfindungsgemäß besonders bevorzugte Mittel sind dadurch gekennzeichnet, daß der Rest R in Formel (X) ausgewählt ist aus Methyl-, Ethyl-, n-Propyl-, iso-Propyl-, n-Butyl-, iso-Butyl-, *tert-*Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäße Mittel bevorzugt, die den Kohlensäuremonoester bzw. das Kohlensäuremonoamid in ganz oder teilweise neutralisierter Form, vorzugsweise in Form seines Alkalimetall-, Ammonium-, Erdalkalimetall- oder Aluminiumsalzes und insbesondere in Form seines Natriumsalzes, enthalten.

Unabhängig davon, ob die erfindungsgemäßen Mittel einen oder mehrere Kohlensäuremonoester und/oder ein oder mehrere Kohlensäuremonoamide enthalten, sind erfindungsgemäße Mittel bevorzugt, die den bzw. die Kohlensäuremonoester und/oder Kohlensäuremonoamide in Mengen von 0,1 bis 20 Gew.%, vorzugsweise von 0,5 bis 18 Gew.%, besonders bevorzugt von 2 bis 15 Gew.% und insbesondere von 5 bis 12 Gew.%, jeweils bezogen auf das gesamte Mittel, enthalten.

Zusätzlich zu Carbonaten und/oder Kohlensäuremonoestern und/oder Kohlensäuremonoamiden oder an deren Stelle können die erfindungsgemäßen Mittel vorzugsweise auch mindestens ein Silylcarbonat und/oder mindestens ein Silylcarbamat enthalten. Diese Substanzen werden nachfolgend detailliert beschrieben.

Erfindungsgemäß bevorzugte Mittel sind dadurch gekennzeichnet, daß sie mindestens ein Silylcarbonat der Formel (XI), 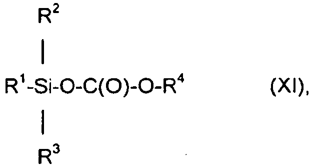 enthalten, in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstiuierte Arylgruppe bzw. einen substituierten oder unsubstituierten Heterocyclus oder für ein Halogen, eine substituierte oder unsubstituierte Hydroxy-, Oxo-, Amino-, Iminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstiuierte Arylgruppe bzw. einen substituierten oder unsubstituierten Heterocyclus steht.

Bevorzugte Reste R¹, R² und R³ in der oben genannten Formel (XI) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße Mittel dadurch gekennzeichnet sind, daß die Reste R¹, R² und R³ in Formel (XI) ausgewählt sind aus Methyl-, Ethyl-, n-Propyl-, iso-Propyl-, n-Butyl-, iso-Butyl-, *tert*-Butylsowie Hydroxymethyl- und Hydroxyethyl-Resten.

Bevorzugte Reste R⁴ in der oben genannten Formel (XI) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Das acide H-Atom des Silylcarbonats (R⁴ = -H in Formel XI) kann auch in neutralisierter Form vorliegen, d.h. es können erfindungsgemäß auch Salze von Silylcarbonaten eingesetzt werden. Hier sind erfindungsgemäße Mittel bevorzugt, die mindestens ein Silylcarbonat in ganz oder teilweise neutralisierter Form, vorzugsweise in Form seines Alkalimetall-, Ammonium-, Erdalkalimetall- oder Aluminiumsalzes und insbesondere in Form seines Natriumsalzes, enthalten.

Zusätzlich zu den Siliylcarbonaten oder an ihrer Stelle können die erfindungsgemäßen Mittel Silylcarbamate enthalten.

Erfindungsgemäß bevorzugte Mittel sind dadurch gekennzeichnet, daß sie ein Silylcarbamat der Formel XII), 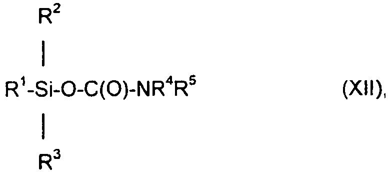 enthalten, in der enthält, in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstiuierte Arylgruppe bzw. einen substituierten oder unsubstituierten Heterocyclus oder für ein Halogen, eine substituierte oder unsubstituierte Hydroxy-, Oxo-, Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstiuierte Arylgruppe bzw. einen substituierten oder unsubstituierten Heterocyclus stehen.

Bevorzugte Reste R¹, R² und R³ in der oben genannten Formel (XII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße Mittel dadurch gekennzeichnet sind, daß die Reste R¹, R² und R³ in Formel (VII) ausgewählt sind aus Methyl-, Ethyl-, n-Propyl-, iso-Propyl-, n-Butyl-, iso-Butyl-, *tert*-Butylsowie Hydroxymethyl- und Hydroxyethyl-Resten.

Bevorzugte Reste R⁴ und R⁵ in der oben genannten Formel (XII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Unabhängig davon, ob die erfindungsgemäßen Mittel ein oder mehrere Silylcarbonate und/oder ein oder mehrere Silylcarbamate enthalten, sind erfindungsgemäße Mittel bevorzugt, die das bzw. die Silylcarbonat(e) und/oder Silylcarbamat(e) in Mengen von 0,1 bis 25 Gew.%, vorzugsweise von 0,5 bis 20 Gew.%, besonders bevorzugt von 1 bis 15 Gew.% und insbesondere von 1,5 bis 10 Gew.%, jeweils bezogen auf das gesamte Mittel, enthalten.

Die erfindungsgemäßen Mittel weisen vorzugsweise einen pH-Wert von 5,0 bis 9,0, vorzugsweise von 5,5 bis 8,0, besonders bevorzugt von 6,0 bis 7,7, weiter bevorzugt von 6,2 bis 7,2 und insbesondere von 6,5 bis 6,8 auf.

Die erfindungsgemäßen Färbe- und/oder Aufhellmittel können zusätzlich mindesten ein Cyanat der Formel M⁺ [OCN]⁻ in der M⁺ für K⁺, Na⁺, Li⁺ oder NH₄⁺ der ½ Ca²⁺, ½Mg²⁺ oder ½ Zn²⁺ steht und/oder mindestens eine Verbindung der Formel H₂N-C(O)-L, in der L für eine durch das Anion ⁻OOH verdrängbare Gruppe steht, enthalten.

Erfindungsgemäß bevorzugte Mittel enthalten daher zusätzlich
- 0,05 bis 5 Gew.-% mindestens eines Cyanats der Formel

   **M⁺ [OCN]⁻**

   in der M⁺ für K⁺, Na⁺, Li⁺ oder NH₄⁺ oder ½ Ca²⁺, ½ Mg²⁺ oder ½ Zn²⁺ steht und/oder
- 0,05 bis 5 Gew.-% mindestens einer Verbindung der Formel

   **H₂N-C(O)-L**
in der L für eine durch das Anion ⁻OOH verdrängbare Gruppe steht.

Als Cyanate können erfindungsgemäß Kaliumcyanat, KOCN, und/oder Natriumcyanat, NaOCN, und/oder Lithiumcyanat, LiOCN, und/oder Ammoniumcyanat, NH₄OCN, und/oder Calciumcyanat, Ca(OCN)₂, und/oder Magnesiumcyanat, Mg(OCN)₂, und/oder Zinkcyanat, Zn(OCN)₂, eingesetzt werden.

Erfindungsgemäß bevorzugte Mittel sind dadurch gekennzeichnet, daß sie 0,075 bis 4 Gew.-%, vorzugsweise 0,1 bis 2,5 Gew.-% und insbesondere 0,2 bis 1 Gew.-% Natriumcyanat und/oder Kaliumcyanat und/oder Ammoniumcyanat enthalten, wobei Natriumcyanat besonders bevorzugt ist.

Anstelle von Cyanaten oder in Kombination mit ihnen können auch Verbindungen der Formel H₂N-C(O)-L, in der L für eine durch das Anion ⁻OOH verdrängbare Gruppe steht, in den erfindungsgemäßen Mitteln enthalten sein. Hier sind erfindungsgemäß bevorzugte Mittel dadurch gekennzeichnet, daß sie 0,075 bis 4 Gew.-%, vorzugsweise 0,1 bis 3,5 Gew.-% und insbesondere 0,2 bis 3 Gew.-% mindestens einer Verbindung der Formel

**H₂N-C(O)-L**

enthalten, in der L ausgewählt ist aus -S-CH₂-COOH, -S-(CH₂)₃SO₃H, -S-(CH₂)₂-NH₂, -OCH₃, -O-(C₆H₄)-SO₃M, -Q⁺, -NH-OH, -O-C(O)-R¹, -O-C(O)-H, -O-CH(OH)₂, -SO₃M, -PH(O)OH, -P(O)(OH)₂, -(NH-Glucosamin)ₙ, -NH-C(O)-NH₂, -NH-NH₂, -NH-CN, -P(OH)(O)-C(O)NH₂, -SCN, -OCN, -S-C(NH)NH₂, 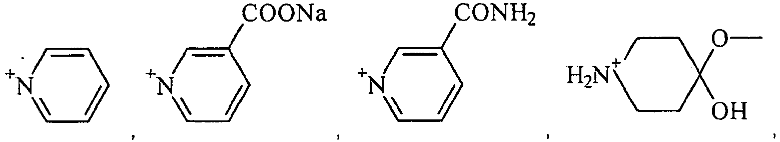 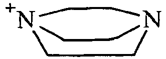 und M für -H, Na, K, NH4 steht, Q ausgewählt ist aus den aus tertiären Aminen gebildeten quartären Ammoniumresten, Heteroarylresten oder nicht-aromatischen Heterocyclen und R¹ für-H, -CH₃, -CH₂CH₃, -(CH₂)₂CH₃, -CH(CH₃)₂ oder eine andere Alkylgruppe steht.

Erfindungsgemäß besonders bevorzugte Mittel enthalten 0,075 bis 4 Gew.-%, vorzugsweise 0,1 bis 3,5 Gew.-% und insbesondere 0,2 bis 3 Gew.-% mindestens einer der nachstehend aufgeführten Verbindungen: H₂N-C(O)-S-CH₂-COOH, H₂N-C(O)-S-(CH₂)₂-NH₂, H₂N-C(O)-S-(CH₂)₃SO₃Na, H₂N-C(O)-S-(CH₂)₃SO₃K, H₂N-C(O)-S-(CH₂)₃SO₃NH₄, H₂N-C(O)-SO₃H, H₂N-C(O)-SO₃Na, H₂N-C(O)-SO₃K, H₂N-C(O)-SO₃NH₄. H₂N-C(O)-O-(C₆H₄)-SO₃H, H₂N-C(O)-O-(C₆H₄)-SO₃Na, H₂N-C(O)-O-(C₆H₄)-SO₃K, H₂N-C(O)-O-(C₆H₄)-SO₃NH₄, H₂N-C(O)-O-C(O)-H, H₂N-C(O)-O-C(O)-CH₃, H₂N-C(O)-O-C(O)-CH₂CH₃, H₂N-C(O)-O-C(O)-(CH₂)₂CH₃, H₂N-C(O)-O-C(O)-CH(CH₃)₂, H₂N-C(O)-OCH₃, H₂N-C(O)-NH-OH, H₂N-C(O)-O-CH(OH)₂, H₂N-C(O)-PH(O)OH, H₂N-C(O)-P(O)(OH)₂, H₂N-C(O)-NH-C(O)-NH₂, H₂N-C(O)-NH-NH₂, H₂N-C(O)-NH-CN, H₂N-C(O)-P(OH)(O)-C(O)NH₂, H₂N-C(O)-SCN, H₂N-C(O)-OCN, H₂N-C(O)-S-C(NH)NH₂, H₂N-C(O)-N(CH₃)₃⁺ Cl⁻, H₂N-C(O)-N(CH₂CH₃)₃⁺ Cl⁻, H₂N-C(O)-N((CH₂)₂CH₃)₃⁺ Cl⁻, H₂N-C(O)-N(CH(CH₃)₂)₃⁺ Cl⁻, H₂N-C(O)-N(C₆H₅)₃⁺ Cl⁻, H₂N-C(O)-N(CH₂-Ph)₃⁺ Cl⁻, H₂N-C(O)-N(CH₃)₃⁺ Br⁻, H₂N-C(O)-N(CH₂CH₃)₃⁺ Br⁻, H₂N-C(O)-N((CH₂)₂CH₃)₃⁺ Br⁻, H₂N-C(O)-N(CH(CH₃)₂)₃⁺ Br⁻, H₂N-C(O)-N(C₆H₅)₃⁺ Br⁻, H₂N-C(O)-N(CH₂-Ph)₃⁺ Br⁻, H₂N-C(O)-N(CH₃)₃⁺ H₃C-OSO₃⁻, H₂N-C(O)-N(CH₂CH₃)₃⁺ H₃C-OSO₃⁻, H₂N-C(O)-N((CH₂)₂CH₃)₃⁺ H₃C-OSO₃⁻, H₂N-C(O)-N(CH(CH₃)₂)₃⁺ H₃C-OSO₃⁻, H₂N-C(O)-N(C₆H₅)₃⁺ H₃C-OSO₃⁻, H₂N-C(O)-N(CH₂-Ph)₃⁺ H₃C-OSO₃⁻, H₂N-C(O)-N(CH₃)₃⁺ H₃C-CH₂-OSO₃⁻, H₂N-C(O)-N(CH₂CH₃)₃⁺ H₃C-CH₂-OSO₃⁻, H₂N-C(O)-N((CH₂)₂CH₃)₃⁺ H₃C-CH₂-OSO₃⁻, H₂N-C(O)-N(CH(CH₃)₂)₃⁺ H₃C-CH₂-OSO₃⁻, H₂N-C(O)-N(C₆H₅)₃⁺ H₃C-CH₂-OSO₃⁻, H₂N-C(O)-N(CH₂-Ph)₃⁺ H₃C-CH₂-OSO₃⁻.

Weiterhin hat es sich als vorteilhaft erwiesen, wenn die erfindungsgemäßen Färbe- und/oder Aufhellmittel nichtionogene grenzflächenaktive Stoffe enthalten. Dabei sind solche grenzflächenaktive Stoffe, die einen HLB-Wert von 5,0 und größer aufweisen, bevorzugt. Für die Definition des HLB-Wertes wird ausdrücklich auf die Ausführungen in Hugo Janistyn, Handbuch der Kosmetika und Riechstoffe, III. Band: Die Körperpflegemittel, 2. Auflage, Dr. Alfred Hüthig Verlag Heidelberg, 1973, Seiten 68-78 und Hugo Janistyn, Taschenbuch der modernen Parfümerie und Kosmetik, 4. Auflage, Wissenschaftliche Verlagsgesellschaft m.b.H. Stuttgart, 1974, Seiten 466-474, sowie die darin zitierten Originalarbeiten Bezug genommen.

Besonders bevorzugte nichtionogene oberflächenaktive Substanzen sind dabei wegen der einfachen Verarbeitbarkeit Substanzen, die kommerziell als Feststoffe oder Flüssigkeiten in reiner Form erhältlich sind. Die Definition für Reinheit bezieht sich in diesem Zusammenhang nicht auf chemisch reine Verbindungen. Vielmehr können, insbesondere wenn es sich um Produkte auf natürlicher Basis handelt, Mischungen verschiedener Homologen eingesetzt werden, beispielsweise mit verschiedenen Alkylkettenlängen, wie sie bei Produkten auf Basis natürlicher Fette und Öle erhalten werden. Auch bei alkoxylierten Produkten liegen üblicherweise Mischungen unterschiedlicher Alkoxylierungsgrade vor. Der Begriff Reinheit bezieht sich in diesem Zusammenhang vielmehr auf die Tatsache, daß die gewählten Substanzen bevorzugt frei von Lösungsmitteln, Stellmittein und anderen Begleitstoffen sein sollen.

Bevorzugte Klassen an nichtionogenen grenzflächenaktiven Stoffen stellen die alkoxylierten Fettalkohole, die alkoxylierten Fettsäuren sowie die Alkylphenole und Alkylphenolalkoxylate dar.Als besonders vorteilhaft haben sich erfindungsgemäße Mittel erwiesen, die nichtionogene grenzflächenaktive Substanzen in Mengen von 1 - 5 Gew.-% enthalten.

Weiterhin können die erfindungsgemäßen Färbe- und/oder Aufhellmittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, kationischen oder nichtionischen Tensiden auszuwählen. Anionische Tenside können dabei ganz besonders bevorzugt sein.

Bevorzugte anionische Tenside sind Alkylsulfate, Ethercarbonsäuresalze mit 10 bis 18 C-Atomen in der Alkylgruppe und bis zu 12 Glykolethergruppen im Molekül wie C₁₂H₂₅-(C₂H₄O)₆-CH₂-COONa sowie insbesondere Salze von gesättigten und speziell ungesättigten C8-C22-Carbonsäuren wie Ölsäure, Stearinsäure, Isostearinsäure und Palmitinsäure.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Ferner können die erfindungsgemäßen Färbe- und/oder Aufhellmittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochiorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel können die Inhaltsstoffe in einem geeigneten wäßrigen, alkoholischen oder wäßrig-alkoholischen Träger enthalten. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, eine pulverförmige oder auch Tabletten-förmige Formulierung bereitzustellen, was für Färbe- und/oder Aufhellmittel bevorzugt ist.

Unter wäßrig-alkoholischen Lösungen sind im Sinne der vorliegenden Erfindung wäßrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel. Bevorzugte erfindungsgemäße Mittel sind dadurch gekennzeichnet, daß sie zusätzlich ein nichtwässriges Lösungsmittel enthalten, wobei besonders bevorzugte erfindungsgemäße Mittel das Lösungsmittel in einer Konzentration von 0,1 - 30 Gewichtsprozent, bevorzugt in einer Konzentration von 1 - 20 Gewichtsprozent, ganz besonders bevorzugt in einer Konzentration von 2 -10 Gewichtsprozent, jeweils bezogen auf das Mittel, enthalten.

Der pH-Wert der erfindungsgemäßen Mittel kann durch geeignete Inhaltstoffe wie Acidifizierungsmittel oder Alkalisierungsmittel in einem weiten Bereich eingestellt werden.

Eine oxidative Färbung der Fasern kann in Gegenwart von Oxidationsfarbstoffvorprodukten grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Dieser Aufhelleffekt kann unabhängig von der Färbemethode gewünscht sein. Die Gegenwart von Oxidationsfarbstoffvorprodukten ist demnach keine zwingende Voraussetzung für einen Einsatz von Oxidationsmitteln in den erfindungsgemäßen Mitteln. Als Oxidationsmittel kommen Persulfate, Chlorite und insbesondere Wasserstoffperoxid oder dessen Anlagerungsprodukte an Harnstoff, Melamin sowie Natriumborat in Frage.

Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Bei einer Anwendung von Oxidationsmitteln wird das eigentliche Färbemittel zweckmäßigerweise unmittelbar vor der Anwendung durch Mischung der Zubereitung des Oxidationsmittels mit der Zubereitung, enthaltend die Piperidon(derivat)e und gegebenenfalls Farbstoffvorprodukte, hergestellt. Das dabei entstehende gebrauchsfertige Haarfärbepräparat sollte bevorzugt einen pH-Wert im Bereich von 6 bis 12 aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann ein erfindungsgemäßes Mittel gegebenenfalls mit zusätzlichen Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Für die Anwendung der erfindungsgemäßen Mittel auf dem Haar werden die Färbe- und/oder Aufhellmittel unmittelbar vor dem Auftragen mit einer Wasserstoffperoxid-Lösung vermischt. Die Konzentration dieser Wasserstoffperoxid-Lösung wird einerseits von den gesetzlichen Vorgaben und andererseits von dem gewünschten Effekt bestimmt; in der Regel werden 6- bis 12prozentige Lösungen in Wasser verwendet. Die Mengenverhältnisse von Färbe- und/oder Aufhellmittel und Wasserstoffperoxid-Lösung liegen dabei üblicherweise im Bereich 1:1 bis 1:2, wobei ein Überschuß an Wasserstoffperoxid-Lösung insbesondere dann gewählt wird, wenn keine zu ausgeprägte Blondierwirkung erwünscht ist.

Erfindungsgemäß bevorzugt ist auch der Einsatz von sogenannten Komplexbildnern. Komplexbilder sind Stoffe, die Metallionen komplexieren können. Bevorzugte Komplexbildner sind sogenannte Chelatkomplexbildner, also Stoffe, die mit Metallionen cyclische Verbindungen bilden, wobei ein einzelner Ligand mehr als eine Koordinationsstelle an einem Zentralatom besetzt, d. h. mind. "zweizähnig" ist. In diesem Falle werden also normalerweise gestreckte Verbindungen durch Komplexbildung über ein Ion zu Ringen geschlossen. Die Zahl der gebundenen Liganden hängt von der Koordinationszahl des zentralen Ions ab.

Erfindungsgemäß bevorzugte Mittel sind dadurch gekennzeichnet, daß sie zusätzlich einen oder mehrere Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine
enthält, wobei bevorzugte Mittel Phosphonate, vorzugsweise Hydroxyalkan- bzw. Aminoalkanphosphonate und insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthalten.

Bevorzugte erfindungsgemäße Mittel werden wasserarm bzw. wasserfrei formuliert. Erfindungsgemäß besonders bevorzugte Mittel sind dadurch gekennzeichnet, daß sie weniger als 5 Gew.-%, vorzugsweise weniger als 2 Gew.-%, besonders bevorzugt weniger als 1 Gew.-% und insbesondere weniger als 0,5 Gew.-% Wasser enthalten, wobei bevorzugte Mittel wasserfrei sind. Der Wassergehalt der Mittel läßt sich beispielsweise mittels Titration nach Karl Fischer bestimmen.

Ein zweiter Gegenstand der vorliegenden Erfindung ist ein Verfahren zum Aufhellen und/oder Färben von Keratinfasern, insbesondere menschlichen Haaren, dadurch gekennzeichnet, daß
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein erfindungsgemäßes Mittel ist.

Die erfindungsgemäßen Mittel können demnach als Einkomponentenmittel (Färbe- und Aufhellmittel M2 bzw. Nachbehandlungsmittel M4), als Zweikomponenten Mittel (M2 + M3) oder als Dreikomponentenmittel (M2 + M3 + M4) formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Färbe- und Aufhellungsverfahren, bei dem die Aufhellcreme und das Oxidationsmittel zunächst getrennt vorliegen, ist dabei bevorzugt. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem erfindungsgemäßen Mittel zu einer homogenen Zusammensetzung vermischt, und diese auf das Haar aufgebracht wird.

In bevorzugten erfindungsgemäßen Verfahren dieser Art enthält die Zusammensetzung auf wäßriger Grundlage bezogen auf ihr Gewicht 1 bis 20 Gew.%, vorzugsweise 2 bis 10 Gew.% und insbesondere 3 bis 6 Gew.% Wasserstoffperoxid, berechnet als 100%iges H₂O₂.

Weiter bevorzugte erfindungsgemäße Verfahren dieser Art sind dadurch gekennzeichnet, daß die Zusammensetzung auf wäßriger Grundlage, enthaltend Wasserstoffperoxid, mit einem erfindungsgemäßen Mittel im Gewichtsverhältnis 1:5 bis 10:1, vorzugsweise 1:2 bis 5:1 und insbesondere 1:2 bis 2:1 zu einer homogenen Zusammensetzung vermischt, und diese auf das Haar aufgebracht wird.

Alternativ kann - wie vorstehend erwähnt - auch ein Dreikomponentensystem zur Anwendung gelangen. Ein weiterer Gegenstand der vorliegenden Erfindung ist daher ein Verfahren zum Färben und Aufhellen von menschlichen Haaren, bei dem eine Zusammensetzung auf wäßriger Grundlage, enthaltend Wasserstoffperoxid, mit einem weiteren Mittel enthaltend vorzugsweise mindestens einen Alkalitätsgeber und/oder direktziehenden Haarfarbstoff und/oder mindestens ein Oxidationsfarbstoffvorprodukt, und einem erfindungsgemäßen Mittel zu einer homogenen Zusammensetzung vermischt, und diese auf das Haar aufgebracht wird.

Bezüglich weiterer bevorzugter Ausführungsformen der erfindungsgemäßen Verfahren gilt mutatis mutandis das zu den erfindungsgemäßen Mitteln Gesagte.

Ein weiterer Gegenstand der vorliegenden Erfindung ist die Verwendung von Piperidonen und/oder Piperidonderivaten in Mitteln zum Färben und/oder Aufhellen keratinischer Fasern.

Besonders bevorzugt dient der Einsatz der Mischungen der Erzielung bestimmter vorteilhafter Effekte. Erfindungsgemäß bevorzugt sind Verwendungen zur
- Steigerung der Aufhell- bzw. Blondierleistung und/oder
- Steigerung der Hautverträglichkeit von Aufhell- bzw. Blondiermitteln.

Bezüglich bevorzugter erfindungsgemäßer Verwendungen gilt mutatis mutandis das zu den bevorzugten Mitteln Ausgeführte.

## Patentansprüche

1. Mittel zum Aufhellen und/oder Färben von Keratinfasern, insbesondere menschlichen Haaren, enthaltend - jeweils bezogen auf sein Gewicht -
a) 0,001 bis 5 Gew.-% eines oder mehrerer Oxidationsfarbstoffvorprodukte und/oder direktziehender Farbstoffe;
b) 0,1 bis 20 Gew.-% mindestens eines Piperidons und/oder Piperidonderivats,
**dadurch gekennzeichnet, dass** es als Piperidonderivat mindestens ein 3-Piperidiniumsalz der Formel (VI) und/oder 4-Piperidiniumsalz der Formel (VII) enthält, 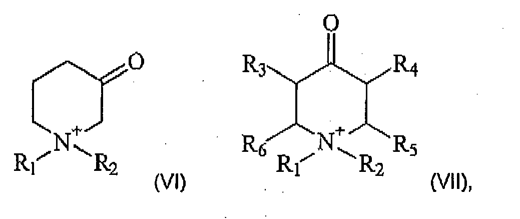 in denen die Reste R¹ bzw. R² bzw, R³ bzw. R⁴ bzw. R⁶ bzw. R⁶ unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, oder anderen Alkylresten, Alkenylresten, gegebenenfalls substituierten Arylresten und bei denen das Gegenion vorzugsweise ausgewählt ist aus Chlorid, Bromid, Methosulfat, Toluolsulfonat.

2. Mittel nach Anspruch 1, **dadurch gekennzeichnet, daß** es 0,1 bis 17,5 Gew.%, vorzugsweise 0,5 bis 15 Gew.-%, besonders bevorzugt 1 bis 12,5 Gew.-% und insbesondere 2,5 bis 10 Gew.-% mindestens einer Verbindung aus der Gruppe
- 2-Piperidon und/oder
- der Derivate von 2-Piperidon, insbesondere der 2-Piperidiniumsalze und/oder
- 3-Piperidon und/oder
- der Derivate von 3-Piperidon, insbesondere der 3-Piperidiniumsalze und/oder
- 4-Piperidon und/oder
- der Derivate von 4-Piperidon, insbesondere der 4-Piperidiniumsalze enthält.

3. Mittel nach einem der Ansprüche 1 bis 2, **dadurch gekennzeichnet, daß** es zusätzlich 0,1 bis 15 Gew.-%, vorzugsweise 0,5 bis 12,5 Gew.-%, besonders bevorzugt 1 bis 10 Gew.% und insbesondere 3 bis 8 Gew.% mindestens einer Imidazolverbindung gemäß Formel (VIII) und/oder deren physiologisch verträglichen Salzen 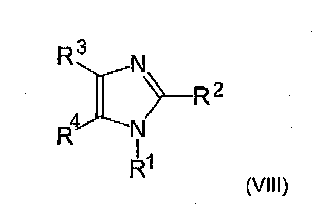 enthält, worin
R¹ steht für ein Wasserstoffatom, eine gegebenenfalls substituierte Arylgruppe oder eine (C₁-C₆)-Alkylgruppe,
R² steht für ein Wasserstoffatom, eine Carboxaldehydgruppe, eine (C₁-C₆)-Alkylgruppe oder eine Nitrogruppe,
R³ steht für ein Wasserstoffatom, eine Carboxy-(C₁-C₆)-alkylgruppe, eine Amino-(C₁-C₆)-alkylgruppe, eine Carboxylgruppe, eine Carboxaldehydgruppe, eine (C₁-C₆)-Alkylgruppe, eine Nitrogruppe, eine 2-Amino-3-hydroxypropylgruppe oder eine Gruppe -CH₂-CH(NH₂)-COOH,
R⁴ steht für ein Wasserstoffatom, eine Carboxaldehydgruppe oder eine Carboxylgruppe.

4. Mittel nach einem der Ansprüche 1 bis 3, **dadurch gekennzeichnet, daß** es zusätzlich 0,1 bis 25 Gew.%, vorzugsweise 0,5 bis 20 Gew.%, besonders bevorzugt 1 bis 15 Gew.% und insbesondere 1,5 bis 10 Gew.% mindestens eines Stoffes aus den Gruppen der
- Carbonate und/oder
- Monoalkylcarbonate (Kohlensäuremonoester) und/oder
- Kohlensäuremonoamide und/oder
- Silylcarbonate und/oder
- Silylcarbamate
enthält.

5. Mittel nach einem der Ansprüche 1 bis 4, **dadurch gekennzeichnet, daß** zusätzlich 0,01 bis 2 Gew.-% mindestens einer festen Peroxoverbindung, die ausgewählt ist aus Ammonium- und Alkalimetall-persulfaten, -peroxomonosulfaten und -peroxidisulfaten enthält, wobei bevorzugte Mittel Peroxidisulfate enthalten, die vorzugsweise ausgewählt sind aus Natriumperoxidisulfat und/oder Kaliumperoxidisulfat und/oder Ammoniumperoxidisulfat und wobei besonders bevorzugte Mittel mindestens zwei verschiedene Peroxidislfate enthalten.

6. Mittel nach einem der Ansprüche 1 bis 5, **dadurch gekennzeichnet, daß** es zusätzlich
- 0,05 bis 5 Gew.-% mindestens eines Cyanats der Formel
M⁺ [OCN]⁻
in der M⁺ für K⁺, Na⁺, Li⁺ oder NH₄⁺ oder ½ Ca²⁺, ½ Mg²⁺ oder ½ Zn²⁺ steht und/oder
- 0,05 bis 5 Gew.-% mindestens einer Verbindung der Formel
H₂N-C(O)-L
in der L für eine durch das Anion ⁻OOH verdrängbare Gruppe steht,
enthält.

7. Mittel nach einem der Ansprüche 1 bis 6, **dadurch gekennzeichnet, daß** es zusätzlich einen oder mehrere Chelatkomplexbildner aus den Gruppen der
(i) Polycarbonsäuren, bei denen die Summe der Carboxyl- und gegebenenfalls Hydroxylgruppen mindestens 5 beträgt,
(ii) stickstoffhaltigen Mono- oder Polycarbonsäuren,
(iii) geminalen Diphosphonsäuren,
(iv) Aminophosphonsäuren,
(v) Phosphonopolycarbonsäuren,
(vi) Cyclodextrine
enthält, wobei bevorzugte Mittel Phosphonate, vorzugsweise Hydroxyalkan- bzw. Aminoalkanphosphonate und Insbesondere 1-Hydroxyethan-1,1-diphosphonat (HEDP) bzw. dessen Di- oder Tetranatriumsalz und/oder Ethylendiamintetramethylenphosphonat (EDTMP) bzw. dessen Hexanatriumsalz und/oder Diethylentriaminpentamethylenphosphonat (DTPMP) bzw. dessen Hepta- oder Octanatriumsalz, enthalten,

8. Mittel nach einem der Ansprüche 1 bis 7, **dadurch gekennzeichnet, daß** es 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid (berechnet als 100%iges H₂O₂) enthält.

9. Mittel nach einem der Ansprüche 1 bis 8, **dadurch gekennzeichnet, daß** es als Oxidationsfarbstoffvorprodukt mindestens eine Entwicklerkomponente enthält, wobei bevorzugte Entwicklerkomponenten ausgewählt sind aus p-Phenylendiamin, p-Toluylendiamin, N,N-Bis-(2-hydroxyethyl)amino-p-phenylendiamin, 1,3-Bis-[(2-hydroxyethyl-4'-amino-phenyl)amino]propan-2-ol, 1,10-Bis-(2',5'-diaminophenyl)-1,4,7,10-tetraoxadecan, 4-Aminophenol, 4-Amino-3-methylphenol, Bis-(5-amino-2-hydroxyphenyl)methan, 2,4,5,6-Tetraaminopyrimidin, 2-Hydroxy-4,5,6-triaminopyrimidin, 4,5-Diamino-1-(2-hydroxyethyl)-pyrazol.

10. Mittel zum Färben und/oder Aufhellen keratinischer Fasern nach einem der Ansprüche 1 bis 9, **dadurch gekennzeichnet, daß** es als Oxidationsfarbstoffvorprodukt mindestens eine Kupplerkomponente enthält, wobei bevorzugte Kupplerkomponenten ausgewählt sind aus Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)-amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)-amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,6-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin.

11. Mittel zum Färben und/oder Aufhellen keratinischer Fasern nach einem der Ansprüche 1 bis 10, **dadurch gekennzeichnet, daß** es mindestens einen direktziehenden Farbstoff enthält, der ausgewählt ist aus Nitrophenylendiaminen, Nitroaminophenolen, Azofarbstoffen, Anthrachinonen oder Indophenolen, vorzugsweise aus der Gruppe der unter den internationalen Bezeichnungen bzw. Handelsnamen bekannten Farbstoffe HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Acid Yellow 87, Basic Orange 31 und Basic Red 51 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)-aminophenol, 2-(2-Hydroxyethyl)-amino-4,6-dinitrophenol, 1-(2'-Hydroxyethyl)amino-4-methyl-2-nitrobenzol, 1-Amino-4-(2-hydroxyethyl)-amino-5-chlor-2-nitrobenzol, 4-Amino-3-nitrophenol, 1-(2-Ureidoethyl)amino-4-nitrobenzol, 4-Amirio-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

12. Verfahren zum Aufhellen und/oder Färben von Keratinfasern, insbesondere menschlichen Haaren, **dadurch gekennzeichnet, daß**
- gewünschtenfalls ein Vorbehandlungsmittel M1 auf die Faser aufgebracht wird, dann
- ein Mittel M2 auf der Faser zur Anwendung kommt, wobei gewünschtenfalls dem Mittel M2 vor der Anwendung ein weiteres Mittel M3 zugegeben wird,
- dieses Mittel M2 nach einer Zeit von 5-30 Minuten von der Faser abgespült wird
- und nach der Behandlung gegebenenfalls ein Nachbehandlungsmittel M4 auf die Faser aufgetragen und nach einer Einwirkzeit von einigen Minuten wieder abgespült wird,
wobei mindestens eines der Mittel M1, M2 oder M3 ein Mittel nach einem der Ansprüche 1 bis 11 ist.

13. Verwendung von Piperidonen und/oder Piperidonderivaten in Mitteln zum Färben und/oder Aufhellen keratinischer Fasern zur Steigerung der Aufhell- bzw. Blondierleistung.

## Claims

1. An agent for lightening and/or coloring keratinic fibers, especially human hair, comprising - each based on its weight -
a) 0.001 to 5 wt % of one or more oxidation dye precursors and/or substantive dyes;
b) 0.1 to 20 wt % of at least one piperidone and/or piperidone derivative,
**characterized in that** it comprises at least one 3-piperidinium salt of the Formula (VI) and/or 4-piperidinium salt of the Formula (VII) 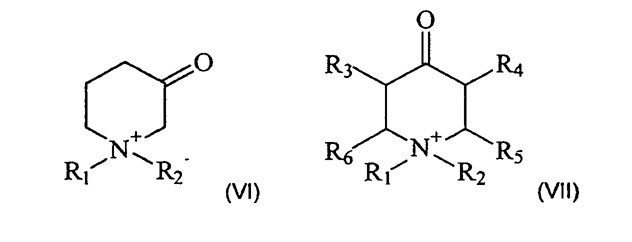 in which the groups R¹, R², R³, R⁴, R⁵ and R⁶ independently of each other are selected from -H, -CH₃, -CH₂CH₃, -(CH₂)₂CH₂, -CH(CH₃)₂,-(CH₂)₃CH₃, -CH(CH₃)CH₂CH₃, -CH₂CH(CH₃)₂, or from other alkyl groups, alkenyl groups, optionally substituted aryl groups and in which the counter ion is preferably selected from chloride, bromide, methosulfate, toluene sulfonate.

2. The agent according to claim 1, **characterized in that** it comprises 0.1 to 17.5 wt %, preferably 0.5 to 15 wt %, particularly preferably 1 to 12.5 wt % and in particular 2.5 to 10 wt % of at least one compound from the group
- 2-piperidone and/or
- the derivatives of 2-piperidone, in particular the 2-piperidinium salts and/or
- 3-piperidone and/or
- the derivatives of 3-piperidone, in particular the 3-piperidinium salts and/or
- 4-piperidone and/or
- the derivatives of 4-piperidone, in particular the 4-piperidinium salts.

3. The agent according to one of claims 1 to 2, **characterised in that** it additionally comprises 0.1 to 15 wt %, preferably 0.5 to 12.5 wt %, particularly preferably 1 to 10 wt % and in particular 3 to 8 wt % of at least one imidazole compound according to Formula (VIII) and/or its physiologically acceptable salts 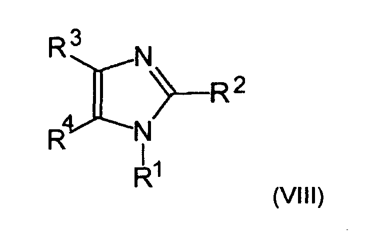 in which,
R¹ stands for a hydrogen atom, an optionally substituted aryl group or a (C₁-C₆) alkyl group,
R² stands for a hydrogen atom, a carboxaldehyde group, a (C₁-C₆) alkyl group or a nitro group,
R³ stands for a hydrogen atom, a carboxy (C₁-C₆) alkyl group, an amino (C₁-C₆) alkyl group, a carboxyl group, a carboxaldehyde group, a (C₁-C₆) alkyl group, a nitro group, a 2-amino-3-hydroxypropyl group or a -CH₂-CH(NH₂)-COOH group,
R⁴ stands for a hydrogen atom, a carboxaldehyde group or a carboxyl group.

4. The agent according to one of claims 1 to 3, **characterized in that** it additionally comprises 0.1 to 25 wt %, preferably 0.5 to 20 wt %, particularly preferably 1 to 15 wt % and in particular 1.5 to 10 wt % of at least one compound from the groups of
- carbonates and/or
- monoalkyl carbonates (monoesters of carbonic acid) and/or
- monoamides of carbonic acid and/or
- silyl carbonates and/or
- silyl carbamates.

5. The agent according to one of claims 1 to 4, **characterized in that** it additionally comprises 0.01 to 2 wt % of at least one solid peroxy compound, selected from ammonium and alkali metal persulfates, peroxy monosulfates and peroxy disulfates, wherein preferred agents comprise peroxy disulfates that are preferably selected from sodium peroxy disulfate and/or potassium peroxy disulfate and/or ammonium peroxy disulfate and wherein particularly preferred agents comprise at least two different peroxy disulfates.

6. The agent according to one of claims 1 to 5, **characterized in that** it additionally comprises
- 0.05 to 5 wt % of at least one cyanate of the Formula
M⁺ [OCN]⁻
in which M⁺ stands for K⁺, Na⁺, Li⁺ or NH₄⁺ or ½ Ca²⁺, ½ Mg²⁺ or ½ Zn²⁺ and/or
- 0.05 to 5 wt % of at least one compound of the Formula
H₂N-C(O)-L
in which L stands for a group that can be displaced by the ⁻OOH anion.

7. The agent according to one of claims 1 to 6, **characterized in that** it additionally comprises one or more chelate complexing agents from the group of the
i. polycarboxylic acids, in which the sum of carboxy and optional hydroxy groups is at least 5,
ii. nitrogen-containing mono- or polycarboxylic acids,
iii. geminal diphosphonic acids,
iv. aminophosphonic acids,
v. phosphonopolycarboxylic acids,
vi. cyclodextrines
wherein preferred agents comprise phosphonates, preferably hydroxyalkane or aminoalkane phosphonates and especially 1-hydroxyethane-1,1-diphosphonate (HEDP) or its di- or tetrasodium salt and/or ethylenediaminetetramethylene phosphonate (EDTMP) or its hexasodium salt and/or diethylenetriaminepentamethylene phosphonate (DTPMP) or its hepta or octasodium salt.

8. The agent according to one of claims 1 to 7, **characterized in that** it comprises 0.5 to 15 wt %, preferably 1 to 12.5 wt %, particularly preferably 2.5 to 10 wt % and in particular 3 to 6 wt % of hydrogen peroxide (calculated as 100% conc. H₂O₂).

9. The agent according to one of claims 1 to 8, **characterized in that** it comprises at least one developer component as the oxidation dye precursor, wherein preferred developer components are selected from p-phenylenediamine, *p*-toluylenediamine, *N,N*-bis-(2-hydroxyethyl)amino-*p-*phenylenediamine, 1,3-bis-[(2-hydroxyethyl-4-aminophenyl)amino]-propane-2-ol, 1,10-bis-(2',5'-diaminophenyl)-1,4,7,10-tetraoxadecane, 4-aminophenol, 4-amino-3-methylphenol, bis-(5-amino-2-hydroxyphenyl)methane, 2,4,5,6-tetraaminopyrimidine, 2-hydroxy-4,5,6-triaminopyrimidine, 4,5-diamino-1-(2-hydroxyethyl)-pyrazole.

10. The agent for coloring and/or lightening keratinic fibers according to one of claims 1 to 9, **characterized in that** it comprises at least one coupler component as the oxidation dye precursor, wherein preferred coupler components are selected from resorcinol, 2-methylresorcinol, 5-methylresorcinol, 2,5-dimethylresorcinol, 4-chlororesorcinol, resorcinol monomethyl ether, 5-aminophenol, 5-amino-2-methylphenol, 5-(2-hydroxyethyl)amino-2-methylphenol, 3-amino-4-chloro-2-methylphenol, 3-amino-2-chloro-6-methylphenol, 3-amino-2,4-dichlorophenol, 2,4-diaminophenoxyethanol, 2-amino-4-(2'-hydroxyethyl)aminoanisole sulfate, 1,3-bis-(2,4-diaminophenoxy)propane, 2-amino-3-hydroxypyridine, 2-methylamino-3-amino-6-methoxypyridine, 2,6-dihydroxy-3,4-dimethylpyridine, 3,5-diamino-2,6-dimethoxypyridine, 1-naphthol, 2-methyl-1-naphthol, 1,5-dihydroxynaphthalene, 2,7-dihydroxynaphthalene, 1-phenyl-3-methylpyrazol-5-one, 2,6-bis-[(2'-hydroxyethyl)amino]-toluene, 4-hydroxyindole, 6-hydroxyindole, 6-hyd roxybenzomorpholine.

11. The agent for coloring and/or lightening keratinic fibers according to one of claims 1 to 10, **characterized in that** it comprises at least one substantive dye that is selected from nitrophenylenediamines, nitroaminophenols, azo dyes, anthraquinones or indophenols, preferably known compounds from the group of the dyes known under the international designations or trade names HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Acid Yellow 87, Basic Orange 31 and Basic Red 51 as well as 1,4-diamino-2-nitrobenzene, 2-amino-4-nitrophenol, 1,4-bis-(β-hydroxyethyl)-amino-2-nitrobenzene, 3-nitro-4-(β-hydroxyethyl)-aminophenol, 2-(2-hydroxyethyl)amino-4,6-dinitrophenol, 1-(2'-hydroxyethyl)amino-4-methyl-2-nitrobenzene, 1-amino-4-(2-hydroxyethyl)-amino-5-chloro-2-nitrobenzene, 4-amino-3-nitrophenol, 1-(2-ureidoethyl)amino-4-nitrobenzene, 4-amino-2-nitrodiphenylamine-2'-carboxylic acid, 6-nitro-1,2,3,4-tetrahydroquinoxaline, 2-hydroxy-1,4-naphthoquinone, picramic acid and its salts, 2-amino-6-chloro-4-nitrophenol, 4-ethylamino-3-nitrobenzoic acid and 2-chloro-6-ethylamino-1-hydroxy-4-nitrobenzene.

12. Method for lightening and/or coloring keratinic fibres, especially human hair, **characterised in that**
- if desired, a pre-treatment agent M1 is deposited onto the fibers, then
- an agent M2 is used on the fibers, wherein if desired an additional agent M3 is added to the agent M2 prior to the application,
- after 5 - 30 minutes this agent M2 is rinsed out of the fibers
- and after the treatment, a post-treatment agent M4 is optionally applied onto the fibers and is rinsed out again after a contact time of some minutes,
wherein at least one of the agents M1, M2 or M3 is an agent according to one of claims 1 to 11.

13. Use of piperidones and/or piperidone derivatives in agents for coloring and/or lightening keratinic fibers so as to increase the lightening or blonding power.

## Revendications

1. Agent pour l'éclaircissement et/ou la teinture de fibres kératiniques, en particulier de cheveux humains, contenant, à chaque fois par rapport à son poids,
a) 0,001 à 5% en poids d'un ou de plusieurs précurseurs de colorants d'oxydation et/ou colorants directs;
b) 0,1 à 20% en poids d'au moins une pipéridone et/ou un dérivé de pipéridone,
**caractérisé en ce qu'**il contient, comme dérivé de pipéridone, au moins un sel de 3-pipéridinium de formule (VI) et/ou un sel de 4-pipéridinium de formule (VII), 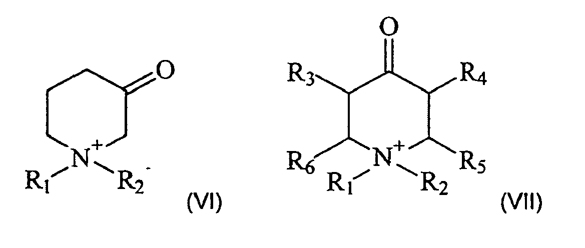 dans lesquelles les résidus R¹ ou R² ou R³ ou R⁴ ou R⁵ ou R⁶ sont choisis, indépendamment les uns des autres, parmi -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂ ou parmi d'autres résidus alkyle, résidus alcényle, résidus aryle le cas échéant substitués et dans lesquels le contre-ion est de préférence choisi parmi le chlorure, le bromure, le méthosulfate, le toluènesulfonate.

2. Agent selon la revendication 1, **caractérisé en ce qu'**il contient 0,1 à 17,5% en poids, de préférence 0,5 à 15% en poids, de manière particulièrement préférée 1 à 12,5% en poids et en particulier 2,5 à 10% en poids d'au moins un composé du groupe formé par
- la 2-pipéridone et/ou
- les dérivés de 2-pipéridone, en particulier les sels de 2-pipéridinium et/ou
- la 3-pipéridone et/ou
- les dérivés de 3-pipéridone, en particulier les sels de 3-pipéridinium et/ou
- la 4-pipéridone et/ou
- les dérivés de 4-pipéridone, en particulier les sels de 4-pipéridinium.

3. Agent selon l'une quelconque des revendications 1 à 2, **caractérisé en ce qu'**il contient en outre 0,1 à 15% en poids, de préférence 0,5 à 12,5% en poids, de manière particulièrement préférée 1 à 10% en poids et en particulier 3 à 8% en poids d'au moins un composé imidazole selon la formule (VIII) et/ou leurs sels physiologiquement acceptables 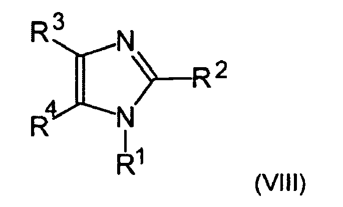 où
R¹ représente un atome d'hydrogène, un groupe aryle le cas échéant substitué ou un groupe (C₁-C₆)-alkyle,
R² représente un atome d'hydrogène, un groupe carboxaldéhyde, un groupe (C₁-C₆)-alkyle ou un groupe nitro,
R³ représente un atome d'hydrogène, un groupe carboxy-(C₁-C₆)-alkyle, un groupe amino-(C₁-C₆)-alkyle, un groupe carboxyle, un groupe carboxaldéhyde, un groupe (C₁-C₆)-alkyle, un groupe nitro, un groupe 2-amino-3-hydroxypropyle ou un groupe -CH₂-CH(NH₂)-COOH,
R⁴ représente un atome d'hydrogène, un groupe carboxaldéhyde ou un groupe carboxyle.

4. Agent selon l'une quelconque des revendications 1 à 3, **caractérisé en ce qu'**il contient en outre 0,1 à 25% en poids, de préférence 0,5 à 20% en poids, de manière particulièrement préférée 1 à 15% en poids et en particulier 1,5 à 10% en poids d'au moins une substance des groupes formés par
- les carbonates et/ou
- les monoalkylcarbonates (monoesters de l'acide carbonique) et/ou
- les monoamides de l'acide carbonique et/ou
- les silylcarbonates et/ou
- les silylcarbamates.

5. Agent selon l'une quelconque des revendications 1 à 4, **caractérisé en ce qu'**il contient en outre 0,01 à 2% en poids d'au moins un composé peroxo solide, qui est choisi parmi les persulfates, les peroxomonosulfates et les bisulfates peroxydés d'ammonium et de métal alcalin, les agents préférés contenant des bisulfates peroxydés, qui sont de préférence choisis parmi le bisulfate peroxydé de sodium et/ou le bisulfate peroxydé de potassium et/ou le bisulfate peroxydé d'ammonium et les agents particulièrement préférés contenant au moins deux bisulfates peroxydés différents.

6. Agent selon l'une quelconque des revendications 1 à 5, **caractérisé en ce qu'**il contient en outre
- 0,05 à 5% en poids d'au moins un cyanate de formule
M⁺[OCN]⁻
dans laquelle M⁺ représente K⁺, Na⁺, Li⁺ ou NH₄⁺ ou ½ Ca²⁺, ½ Mg²⁺ ou ½ Zn²⁺ et/ou
- 0,05 à 5% en poids d'au moins un composé de formule
H₂N-C(O)-L
dans laquelle L représente un groupe pouvant être déplacé par l'anion ⁻OOH.

7. Agent selon l'une quelconque des revendications 1 à 6, **caractérisé en ce qu'**il contient en outre un ou plusieurs complexants chélates des groupes formés par
i. les acides polycarboxyliques dans lesquels la somme des groupes carboxyle et le cas échéant hydroxyle est d'au moins 5,
ii. les acides monocarboxyliques ou polycarboxyliques contenant de l'azote,
iii. les acides diphosphoniques géminaux,
iv. les acides aminophosphoniques,
v. les acides phosphonopolycarboxyliques,
vi. les cyclodextrines,
les agents préférés contenant des phosphonates, de préférence des hydroxyalcanephosphonates ou des aminoalcanephosphonates et en particulier le 1-hydroxyéthane-1,1-diphosphonate (HEDP) ou son sel disodique ou tétrasodique et/ou l'éthylènediaminetétraméthylènephosphonate (EDTMP) ou son sel hexasodique et/ou le diéthylènetriaminepentaméthylènephosphonate (DTPMP) ou son sel heptasodique ou octasodique.

8. Agent selon l'une quelconque des revendications 1 à 7, **caractérisé en ce qu'**il contient 0,5 à 15% en poids, de préférence 1 à 12,5% en poids, de manière particulièrement préférée 2,5 à 10% en poids et en particulier 3 à 6% en poids de peroxyde d'hydrogène (calculé comme H₂O₂ à 100%).

9. Agent selon l'une quelconque des revendications 1 à 8, **caractérisé en ce qu'**il contient comme précurseur de colorant par oxydation au moins un composé développeur, les composants développeurs préférés étant choisis parmi la p-phénylènediamine, la p-toluylènediamine, la N,N-bis-(2-hydroxyéthyl)amino-p-phénylènediamine, le 1,3-bis-[(2-hydroxyéthyl-4'-aminophényl)amino]propan-2-ol, le 1,10-bis-(2',5'-diaminophényl)-1,4,7,10-tétraoxadécane, le 4-aminophénol, le 4-amino-3-méthylphénol, le bis-(5-amino-2-hydroxyphényl)méthane, la 2,4,5,6-tétraaminopyrimidine, la 2-hydroxy-4,5,6-triaminopyrimidine, le 4,5-diamino-1-(2-hydroxyéthyl)-pyrazole.

10. Agent pour la teinture et/ou l'éclaircissement de fibres kératiniques selon l'une quelconque des revendications 1 à 9, **caractérisé en ce qu'**il contient, comme précurseur de colorant par oxydation, au moins un composant de couplage, les composants de couplage préférés étant choisis parmi le résorcinol, le 2-méthylrésorcinol, le 5-méthylrésorcinol, le 2,5-diméthylrésorcinol, le 4-chlororésorcinol, le résorcinolmonométhyléther, le 5-aminophénol, le 5-amino-2-méthylphénol, le 5-(2-hydroxyéthyl)-amino-2-méthylphénol, le 3-amino-4-chloro-2-méthylphénol, le 3-amino-2-chloro-6-méthylphénol, le 3-amino-2,4-dichlorophénol, le 2,4-diaminophénoxyéthanol, le sulfate de 2-amino-4-(2'-hydroxyéthyl)-aminoanisole, le 1,3-bis-(2,4-diaminophénoxy)propane, la 2-amino-3-hydroxypyridine, la 2-méthylamino-3-amino-6-méthoxypyridine, la 2,6-dihydroxy-3,4-diméthylpyridine, la 3,5-diamino-2,6-diméthoxypyridine, le 1-naphtol, le 2-méthyl-1-naphtol, le 1,5-dihydroxynaphtalène, le 2,7-dihydroxynaphtalène, la 1-phényl-3-méthylpyrazol-5-one, le 2,6-bis-[(2'-hydroxyéthyl)amino]-toluène, le 4-hydroxyindole, le 6-hydroxyindole, la 6-hydroxybenzomorpholine.

11. Agent pour la teinture et/ou l'éclaircissement de fibres kératiniques selon l'une quelconque des revendications 1 à 10, **caractérisé en ce qu'**il contient au moins un colorant direct, qui est choisi parmi les nitrophénylènediamines, les nitroaminophénols, les colorants azo, les anthraquinones ou les indophénols, de préférence dans le groupe des colorants connus sous les dénominations internationales ou commerciales HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, Acid Black 52, Acid Yellow 87, Basic Orange 31 et Basic Red 51 ainsi que le 1,4-diamino-2-nitrobenzène, le 2-amino-4-nitrophénol, 1,4-bis-(ß-hydroxyéthyl)amino-2-nitrobenzène, le 3-nitro-4-(ß-hydroxyéthyl)-aminophénol, le 2-(2-hydroxyéthyl)-amino-4,6-dinitrophénol, le 1-(2'-hydroxyéthyl)amino-4-méthyl-2-nitrobenzène, le 1-amino-4-(2-hydroxyéthyl)-amino-5-chloro-2-nitrobenzène, le 4-amino-3-nitrophénol, le 1-(2-uréidoéthyl)amino-4-nitrobenzène, l'acide 4-amino-2-nitrodiphénylamine-2'-carboxylique, la 6-nitro-1,2,3,4-tétrahydroquinoxaline, la 2-hydroxy-1,4-naphtoquinone, l'acide picramique et ses sels, le 2-amino-6-chloro-4-nitrophénol, l'acide 4-éthylamino-3-nitrobenzoïque et le 2-chloro-6-éthylamino-1-hydroxy-4-nitrobenzène.

12. Procédé pour l'éclaircissement et/ou la teinture de fibres kératiniques, en particulier de cheveux humains, **caractérisé en ce que**
- on applique, si souhaité, un agent de prétraitement M1 sur les fibres, puis
- on utilise un agent M2 sur les fibres, l'agent M2 étant si souhaité mélangé avant l'utilisation avec un autre agent M3,
- cet agent M2 est éliminé des fibres par rinçage après un laps de temps de 5-30 minutes,
- et on applique après le traitement le cas échéant un agent de post-traitement M4 sur les fibres qui est à nouveau éliminé par rinçage après un temps d'action de quelques minutes,
au moins un des agents M1, M2 ou M3 étant un agent selon l'une quelconque des revendications 1 à 11.

13. Utilisation de pipéridones et/ou de dérivés de pipéridone dans des agents pour la teinture et/ou l'éclaircissement de fibres kératiniques en vue d'augmenter la puissance d'éclaircissement ou de décoloration.
